(19) Europäisches Patentamt
European Patent Office
Office européen des brevets

(11) Publication number: **0 046 710**
**B1**

(12) # EUROPEAN PATENT SPECIFICATION

(45) Date of publication of patent specification: **13.06.84**

(21) Application number: **81401327.2**

(22) Date of filing: **21.08.81**

(51) Int. Cl.[3]: **C 07 C 121/42,**
C 07 C 121/75,
C 07 C 121/80,
C 07 C 120/00

(54) **Preparation of aminoacetonitrile derivatives.**

(30) Priority: **23.08.80 GB 8027503**
**26.11.80 US 210499**

(43) Date of publication of application:
**03.03.82 Bulletin 82/9**

(45) Publication of the grant of the patent:
**13.06.84 Bulletin 84/24**

(84) Designated Contracting States:
**AT BE CH DE FR IT LI NL SE**

(56) References cited:
**US - A - 3 422 132**

(73) Proprietor: **MERRELL TORAUDE ET COMPAGNIE**
**16 Rue d'Ankara**
**F-67084 Strasbourg (FR)**

(72) Inventor: **Gerhart, Fritz**
**Rheinauerstrasse 14**
**D-7640 Kehl-Leutesheim (DE)**

(74) Representative: **Burford, Anthony Frederick et al,**
**W.H. Beck, Greener & Co. 7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 046 710 B1

## Description

Field of the invention

The invention relates to 2-fluorinated methyl aminoacetonitriles which are useful intermediates in the preparation of pharmaceutically active compounds especially 2-fluorinated methyl aminoacetic acid derivatives and provides a method of preparing said intermediates.

Summary of the invention

2-Fluorinated methyl aminoacetonitriles are prepared by treating a corresponding fluorinated methyl ketimine magnesium halide with hydrogen cyanide or with an alkali metal cyanide or ammonium cyanide and a proton source. The aminoacetonitriles readily can be converted to the corresponding 2-fluorinated methyl aminoacetic acid derivatives. Said aminoacetic acid derivatives are useful compounds in that many have pharmacological or biological activity whilst others are intermediates in the preparation of compounds having pharmacological or other uses.

Background of the invention

A number of classes of 2-fluorinated methyl aminoacetic acid derivatives have been reported to have useful pharmacological activity especially in the field of enzyme inhibition (see, for example, UK Patent Specifications Nos. 2001309A, 2001626A, 2001960A and 2005659A and European Patent Specification No. 0000034A). Further, additional classes of 2-fluorinated methyl aminoacetic acid derivatives are the subject of various co-pending UK Foreign Patent Applications in the name of Merrell Toraude et Compagnie.

It has been disclosed in USA Patent Application No. 33719 filed 26th April 1979 that certain of the monofluoromethyl aminoacetic acid derivatives disclosed in UK Patent Specification No. 2005659A can be prepared by acid hydrolysis of a corresponding monofluoromethyl benzyl ketimine magnesium halide, conversion of the resultant ketone to the corresponding aminoacetonitrile by a Strecker reaction using sodium cyanide and an ammonium salt such as ammonium chloride, and hydrolysis of the amino-nitrile to the corresponding aminoacetic acid derivative.

Surprisingly, it has now been found that said monofluoromethyl aminoacetonitriles can be prepared directly from the monofluoromethyl benzyl ketimine magnesium halides by treatment with hydrogen cyanide or with an alkali metal cyanide or ammonium cyanide and a proton source. This reaction is novel and further also has application to the preparation of other 2-fluorinated methyl aminoacetonitriles.

Detailed description of the invention

Fluorinated methyl ketimine magnesium halides are treated with a cyanide in the presence of a proton source to produce 2-fluorinated methyl aminoacetonitriles. The proton source and cyanide both can be provided by hydrogen cyanide. Alternatively, the reaction can be carried out using an alkali metal cyanide or ammonium cyanide and a proton source but care is required to control the acidity to prevent formation of the corresponding ketone.

The process requires one equivalent of the cyanide and two equivalents of the proton source. Hydrogen cyanide by itself can serve as both the proton source and the cyanide source, provided two equivalents are employed. Alternatively, the cyanide source can be an alkali metal cyanide (such as sodium cyanide or potassium cyanide), or ammonium cyanide, and the proton source can be any organic or inorganic substance of sufficient acidity to form the imine from the ketimine magnesium halide. Weak acids, such as a water-soluble ammonium salt of a strong acid, are a preferred proton source. Ammonium chloride is especially preferred. Although strong organic or inorganic acids can be employed as the proton source, care must be taken to control the amount of acid to prevent or limit ketone formation arising from acid hydrolysis of the imine. Weak acids are preferred because hydrolysis of the imine to the corresponding ketone does not occur to any substantial extent and an excess of the weak acid can be used.

The reaction medium can be any protic or aprotic inert solvent or mixture of such solvents. By "inert solvent" is meant a solvent which is capable of substantially dissolving the reactants and which is not deleterious to the formation of the desired product. An aqueous medium is preferred. By "aqueous medium" is meant either water substantially free of other solvents or water in the presence of other compatible miscible or immiscible solvents. A suitable protic solvent can also function as the proton source.

The process can preferably be carried out at a temperature varying from about 0°C to room temperature and a reaction time of about 30 minutes to about one hour. The temperature and time are not critical. Other temperatures and times may be employed depending upon the particular reactants and/or medium employed. The selection of the optimum reaction conditions and the reaction medium is within the skill of the art.

The ketimine salt can be a bromide, chloride or iodide but the chlorides and, especially, bromides are preferred. Having regard to the classes of pharmacologically active 2-fluorinated methyl aminoacetic acid derivatives referred to hereinbefore, it is preferred that the 2-fluorinated methyl group of the

ketimine salt is a 2-monofluoromethyl or 2-difluoromethyl group.

In a preferred embodiment of the invention, there is provided a process of preparing a 2-fluorinated methyl aminoacetonitrile of the following general Formula I:—

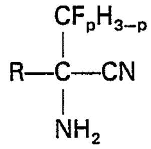

Formula I

wherein:—

R represents an organic group which is inert under the process conditions; and

*p* represents 1 or 2;

which comprises treating a ketimine magnesium halide of the following general Formula II:—

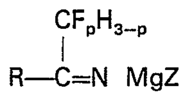

Formula II

wherein:

R and *p* are as defined above; and

Z represents bromine, chlorine or iodine;

with hydrogen cyanide or with an alkali metal cyanide or ammonium cyanide and a proton source.

The organic group R can be, for example, an alkyl or alkenyl group optionally substituted by substituents which are inert under the process conditions. Examples of said substituents are alkoxy, e.g. methoxy; phenylalkoxy, e.g. benzyloxy, diphenylmethoxy and triphenylmethoxy; alkenyloxy, e.g. allyloxy; phenyl; alkoxyphenyl, and benzyloxyphenyl, e.g. those alkoxyphenyl and benzyloxyphenyl groups specified in UK Patent Specification No. 2005659A or European Patent Application No. 0000034.

In an especially preferred embodiment of the invention, there is provided a process for preparing a compound of the formula:

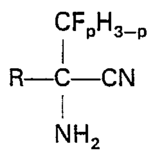

I

wherein:

*p* is 1 or 2

and R is:

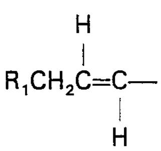 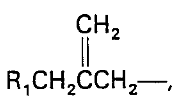, $R_2OCH_2CH_2CH_2$—, $R_2OCH_2CH_2CH_2CH_2$—, $R_3CH_2$—, $CH_2{=}CHCH_2$—,

$CH_2{=}CHCH_2CH_2$—, or $CH_3CH{=}CHCH_2CH_2$—

wherein:

$R_1$ is hydrogen, methoxy, benzyloxy, diphenylmethoxy, triphenylmethoxy, or allyloxy;

$R_2$ is methyl, benzyl, diphenylmethyl, triphenylmethyl, or allyl; and

$R_3$ is a substituted-phenyl group of the formula:

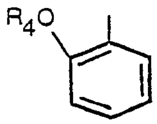 , 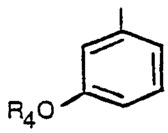 , 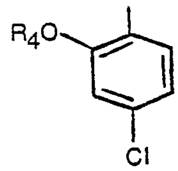 , 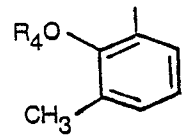 , 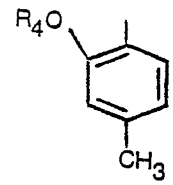 ,

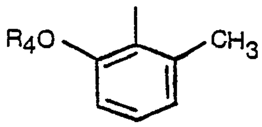 , 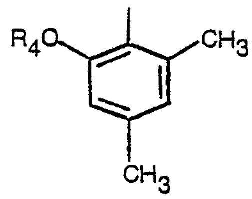 , 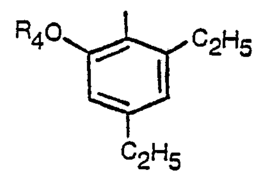 ,

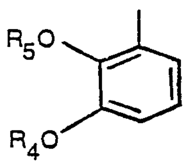 , 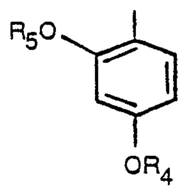 , 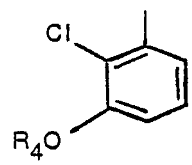 , 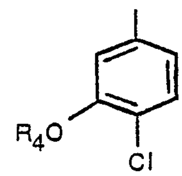 ,

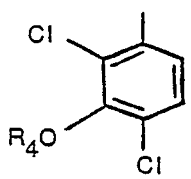 , 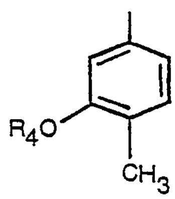 , 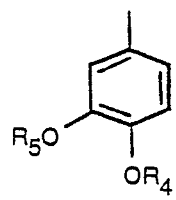 , 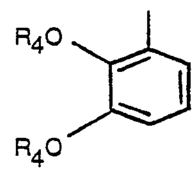 ,

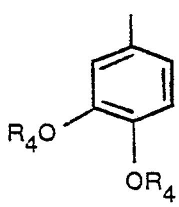 , 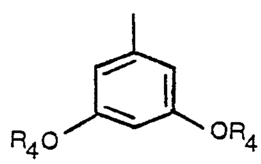 , 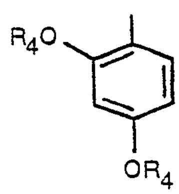 , 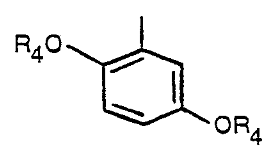 ,

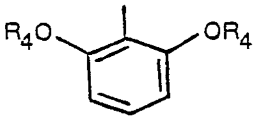 , 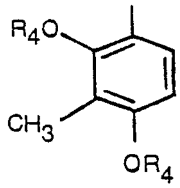 , 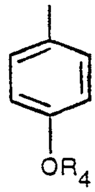 , 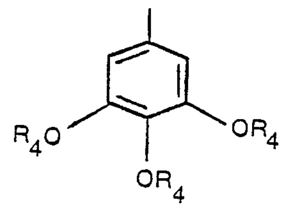 , or

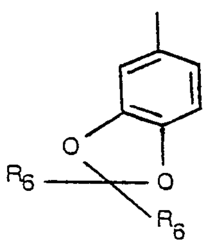 ;

wherein $R_4$ is $C_1$—$C_8$ alkyl, $R_5$ is benzyl, and $R_6$ is hydrogen or methyl; with the proviso that when R is $R_3CH_2$— wherein $R_3$ is

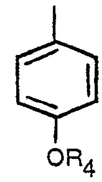

*p* cannot be 2;
which comprises treating a ketimine magnesium halide of the formula:

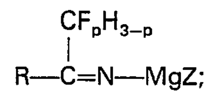

II

wherein R and *p* have the meanings hereinabove defined and Z is chloride, bromide, or iodide; with hydrogen cyanide or with an alkali metal cyanide or ammonium cyanide and a proton source.

It is believed that the process proceeds via formation of the corresponding imine of the formula:

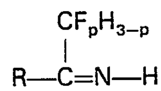

III

wherein R and *p* have the meanings hereinabove defined. The imine (III) can then form the corresponding immonium ion which then reacts with the cyanide ion, or it can add hydrogen cyanide directly. Whatever the exact mechanism, the over-all process can be depicted as shown below:

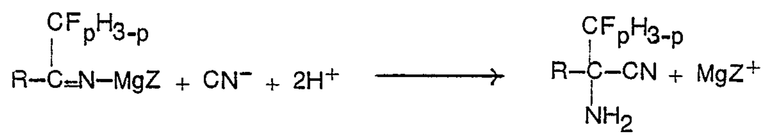

The ketimine magnesium halides of Formula II can be made by reaction of a Grignard reagent of the formula:

RMgZ

IV

wherein R and Z have the meanings hereinbefore defined, with fluoroacetonitrile ($CH_2FCN$) or difluoroacetonitrile ($CHF_2CN$). The Grignard reagent (IV) can be made in known manner from the corresponding organohalide (R—Z) by reaction with magnesium. The reaction of the Grignard reagent with fluoroacetonitrile or difluoroacetonitrile is carried out in a conventional manner in an aprotic solvent (for example, tetrahydrofuran or diethyl ether), at a temperature of from about —20° to —70°C, preferably —20° to —40°C. A reaction time of about 10 minutes to 12 hours (preferably 10 minutes to one hour) is employed.

The ketimine magnesium halide formed by the reaction of the Grignard reagent with fluoroacetonitrile or difluoroacetonitrile need not be isolated from its reaction mixture. Conveniently, the reaction mixture containing the ketimine magnesium halide can be added directly to the reaction medium employed for preparation of the aminonitrile. Using this technique, whatever solvent is employed for preparing the ketimine magnesium halide will be incorporated into the medium utilized for preparation of the aminonitrile. A preferred method is to prepare the ketimine magnesium halide in tetrahydrofuran (THF) and to add the reaction mixture thus formed directly to a water solution of hydrogen cyanide or to an acidic water solution of an alkali metal cyanide or of ammonium cyanide. The use of ammonium chloride as the acid is particularly preferred. Using this technique it is preferable to employ a water-miscible solvent (e.g. tetrahydrofuran) for preparation of the ketimine magnesium halide; but immiscible solvents, such as diethyl ether, can also be used. When difluoroacetonitrile is employed as the reactant for the formation of the ketimine magnesium halide from a benzylic Grignard reagent (i.e. $R_3CH_2MgX$), a mixture of the desired ketimine salt and a ketimine salt side product (formed by *ortho*-

attack by the nitrile at the benzene ring) may be produced. The desired amino-nitrile can be purified by conventional methods, such as chromatography.

The organo-halides (R—Z) employed for preparing the Grignard reagents (RMgZ) are either known compounds or can be prepared by known methods, or obvious variations thereof, from known starting materials.

The term "alkyl" when used herein means a saturated branched or straight-chain hydrocarbon radical. Examples of $C_1$—$C_8$ alkyl groups are methyl, ethyl, *n*-propyl, *i*-propyl, n-butyl, *i*-butyl, t-butyl, n-pentyl, n-hexyl, n-heptyl, and n-octyl. Methyl is preferred.

The fluorinated amino-nitriles of Formula I are useful as intermediates for preparing various pharmacologically or biologically active fluorinated amino acid products:

When R in Formula I is

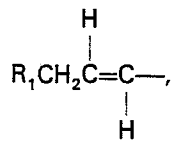

the products are the fluorinated *trans*-dehydro-ornithine derivatives of Formula V:

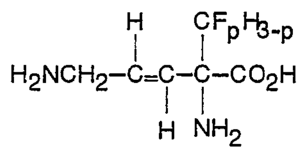

V

wherein *p* is 1 or 2.

When R in Formula I is $R_2OCH_2CH_2CH_2$—, the products are the fluorinated ornithine derivatives of Formula VI:

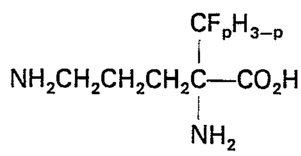

VI

wherein *p* is 1 or 2.

When R in formula I is —$CH_2R_3$, the products are the fluorinated 2-amino-3-(substituted)phenyl propionic acid derivatives of Formula VII:

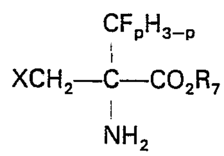

VII

wherein $R_7$ is hydrogen or $C_1$—$C_8$ alkyl, *p* is 1 or 2, and X is a substituted phenyl group of the formula:

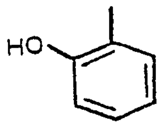 , 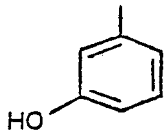 , 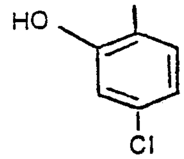 , 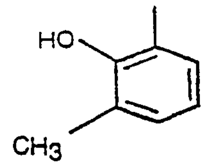 ,

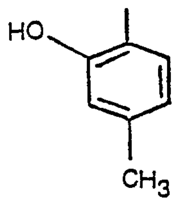 , 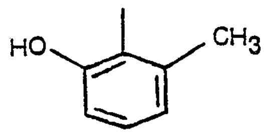 , 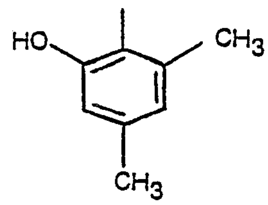 ,

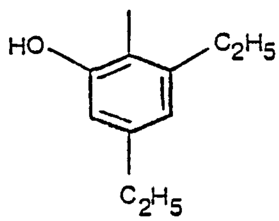 , 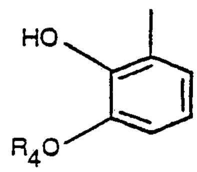 , 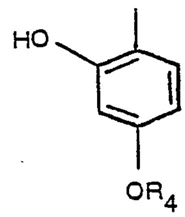 , 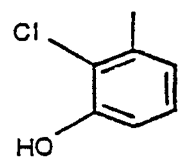

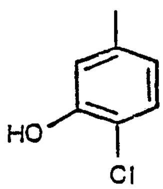 , 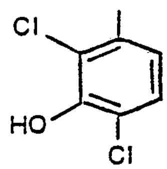 , 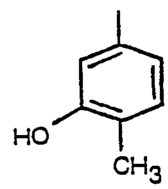 , 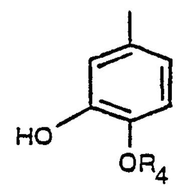 ,

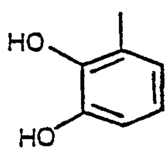 , 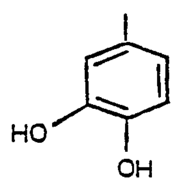 , 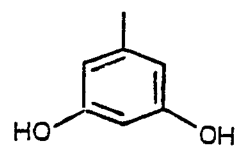 , 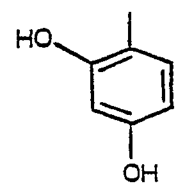 ,

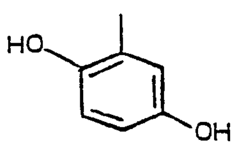 , 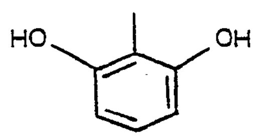 , 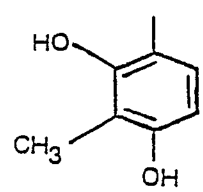 ,

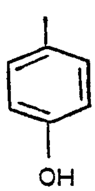 or 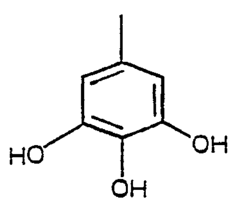 ,

provided that when X is

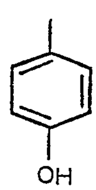

*p* cannot be 2.

When R in Formula I is $R_2OCH_2CH_2CH_2CH_2$—, the products are the fluorinated lysine derivatives of Formula VIII:

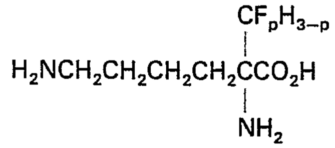

VII

wherein *p* is 1 or 2.

When R in Formula I is

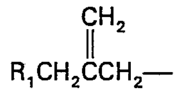

the products are the fluorinated γ-methylene ornithine derivatives of Formula IX:

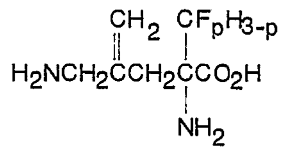

IX

wherein *p* is 1 or 2;
or the fluorinated γ-oxo ornithine derivatives of Formula X:

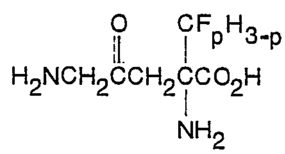

X

wherein *p* is 1 or 2;
or the fluorinated histidine derivatives of Formula XI:

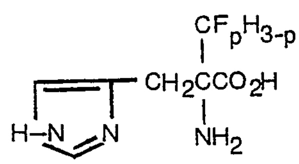

XI

wherein *p* is 1 or 2.

When R in Formula I is

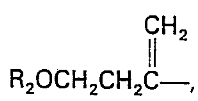

the products are the fluorinated β-methylene ornithine derivatives of Formula XII:

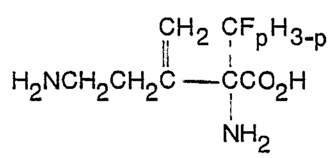

XII

wherein *p* is 1 or 2;
or the β-oxo fluorinated ornithine derivatives of Formula XIII:

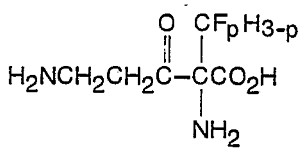

XIII

wherein $p$ is 1 or 2.

When R in Formula I is $CH_2{=}CH{-}CH_2{-}$, the products are the fluorinated $\gamma$-oxo ornithine derivatives of Formula X or the histidine derivatives of Formula XI, wherein $p$ is 1 or 2.

The fluorinated *trans*-dehydroornithine derivatives of Formula V can be prepared using conventional procedures according to the reaction sequence (Scheme A) depicted below:

Scheme A

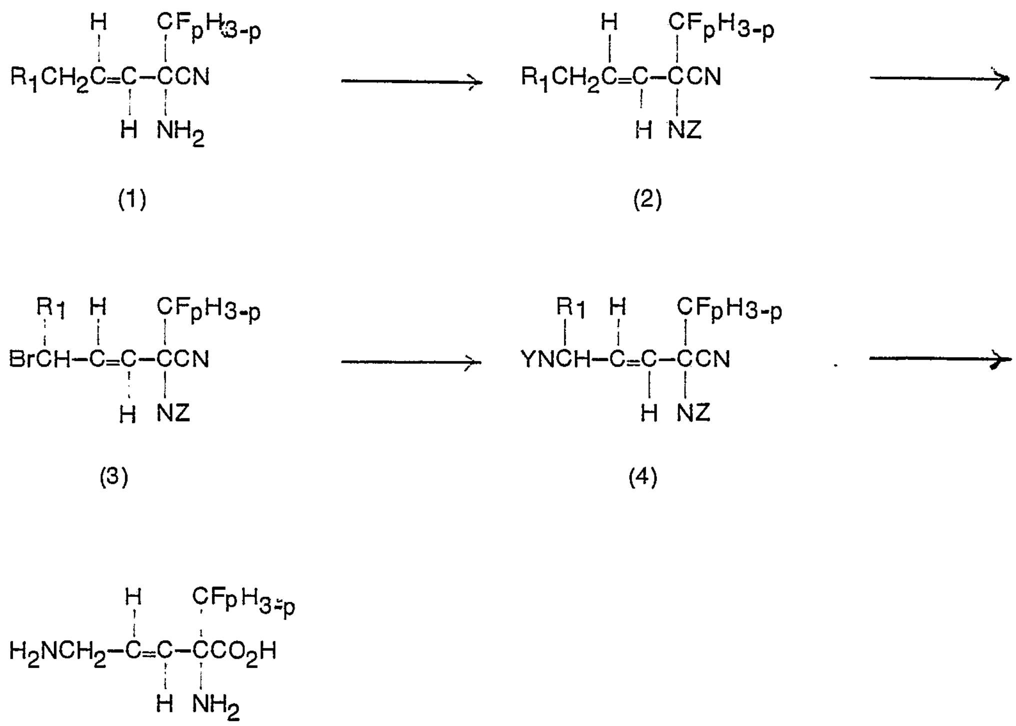

(1) (2)

(3) (4)

(V)

In Scheme A, $R_1$ is hydrogen, $p$ is 1 or 2, NZ is the trifluoroacetamido group or the phthalimido group, or other suitable protecting group for a primary amine, and YN is the hexamethylenetetrammonium group or the phthalimido group.

In the first step of Scheme A, the fluorinated aminonitrile intermediate (1) is treated with trifluoroacetic anhydride or phthaloyldichloride to form the N-protected amino-nitrile (2), which then undergoes allylic bromination with N-bromosuccinimide in the presence of benzoylperoxide to afford the bromo N-protected aminonitrile (3). Treatment of (3) with hexamethylenetetramine or an alkali metal phthalimide provides the corresponding N,N'-diprotected diamino-nitrile derivative (4) which is heated with conc. hydrochloric acid at 100°C to simultaneously hydrolyze the nitrile group and remove the amino protecting groups, thus affording the final product (V).

The products of Formula VI can be prepared using conventional procedures according to the reaction sequence (Scheme B) depicted below.

Scheme B

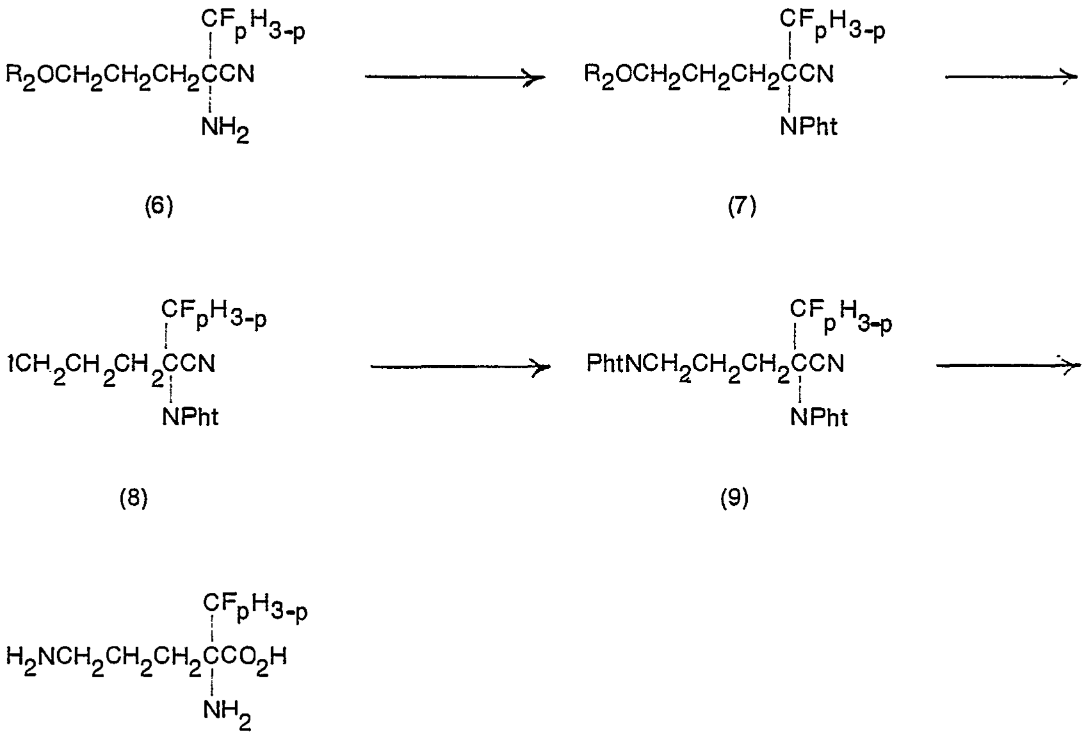

(6) (7) (8) (9) (VI)

In Scheme B, $R_2$ and *p* have the meanings hereinbefore defined, and PhtN or NPht means the phthalimido group, which has the following structural formula:

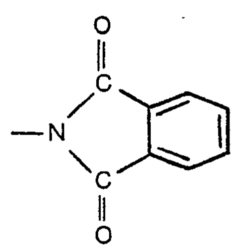

In the first step of Scheme B, the fluorinated aminonitrile intermediate (6) is treated with phthaloyldichloride to form the N-protected amino-nitrile (7). The alkoxy group ($OR_2$) of the N-protected amino-nitrile intermediate (7) is then replaced by iodine by conventional methods. In one method, the intermediate (7) is treated with three equivalents of trimethylsilyliodide (TMSI) which affords the iodo intermediate (8) directly. In another method, the intermediate (7) is treated with two equivalents of TMSI and water under which conditions the alkoxy group of intermediate (7) is cleaved to the corresponding free hydroxy group. The hydroxy intermediate thus formed is then treated with methanesulfonyl chloride to give the corresponding methanesulfonyloxy intermediate which upon treatment with sodium iodide provides the iodo intermediate (8). Treatment of the iodo intermediate (8) with an alkali metal phthalimide gives the N,N′-diprotected diaminonitrile (9) which is heated with concentrated mineral acid (e.g. hydrochloric acid) to simultaneously hydrolyze the nitrile group and remove the N-protecting groups, thus affording the final product (VI).

The method of Scheme B can also be employed to prepare the fluorinated lysine derivatives of Formula VIII or the fluorinated β-methylene ornithine derivatives of Formula XII using as starting materials for the reaction sequence the fluorinated amino-nitriles of Formula (10) and (11), respectively:

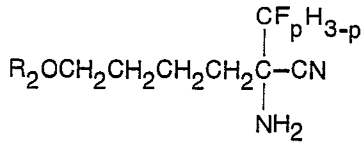

(10)

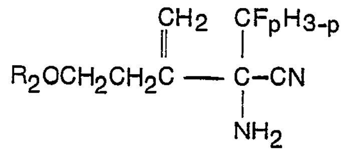

(11)

wherein $R_2$ and *p* have the meanings hereinbefore defined.

A modification of the method depicted in Scheme B can be employed to prepare the fluorinated *trans*-dehydro ornithine derivatives of Formula V or the γ-methylene derivatives of Formula IX from the respective fluorinated amino-nitrile starting materials of Formula (12) and (13), respectively:

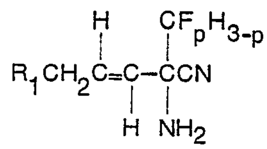

(12)

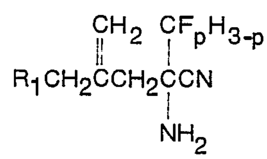

(13)

wherein $R_1$ is methoxy, benzyloxy, diphenylmethoxy, triphenyloxy, or allyloxy, and *p* is 1 or 2. $R_1$ is preferably methoxy. In this modification, instead of treating the N-protected amino-nitrile with TMSI, the N-protected amino-nitrile is treated with boron tribromide to form the corresponding bromo intermediate. The bromo intermediate is then used in place of the iodo intermediate in the reaction sequence of Scheme B to prepare the N,N′-diprotected diamino nitrile which is finally hydrolyzed to the desired product of Formula V or IX.

The fluorinated γ-oxo ornithine derivatives of Formula X can be prepared by conventional procedures according to the reaction sequence (Scheme C) depicted below:

Scheme C

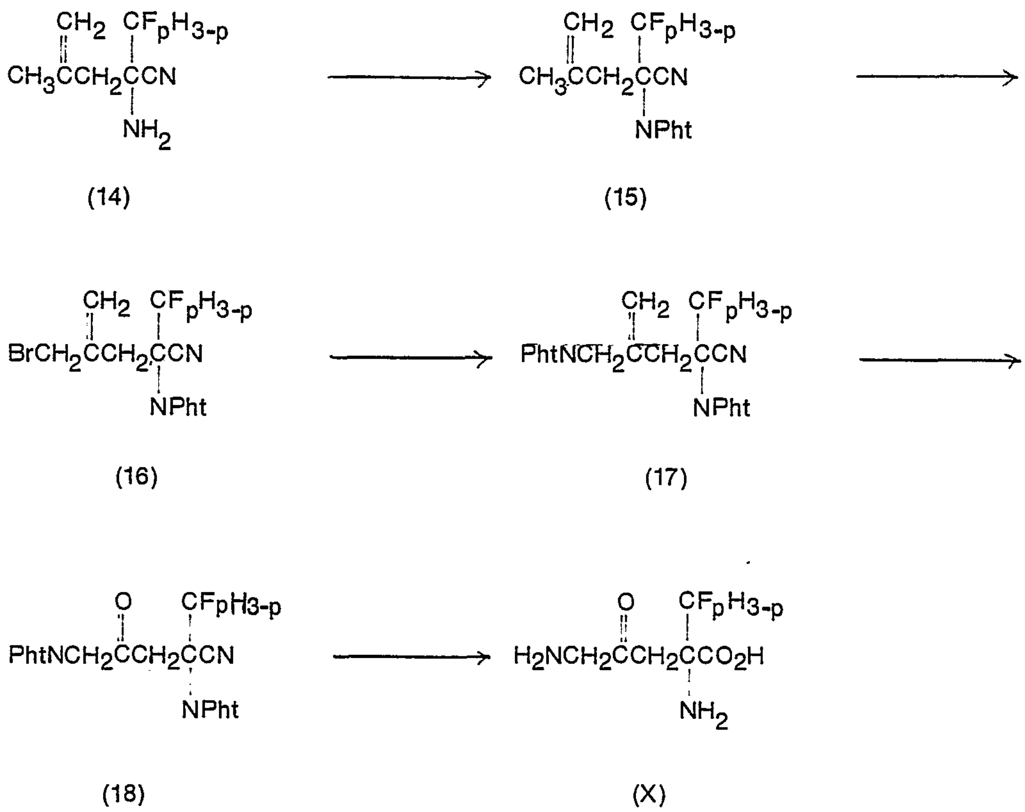

(14) (15) (16) (17) (18) (X)

In Scheme C, PhtN, NPht, and *p* have the meanings as employed in Scheme B.

In the first step of Scheme C, the fluorinated aminonitrile (14) is treated with phthaloyldichloride to afford the N-protected amino-nitrile (15). The N-protected amino-nitrile (15) is then brominated with N-bromo-succinimide in the presence of benzoyl peroxide to give the bromo N-protected amino-nitrile (16). Treatment with potassium phthalimide provides the N,N'-diprotected diamino-nitrile (17) which is oxidized with ozone to afford the $\gamma$-oxo intermediate (18). Treatment of this intermediate with concentrated hydrochloric acid at 100°C removes both amino protecting groups and hydrolyzes the nitrile function to give the desired fluorinated $\gamma$-oxo ornithine derivative (X).

The fluorinated $\gamma$-oxo ornithine derivatives of Formula X can also be prepared by the reaction sequence (Scheme D) shown below:

Scheme D

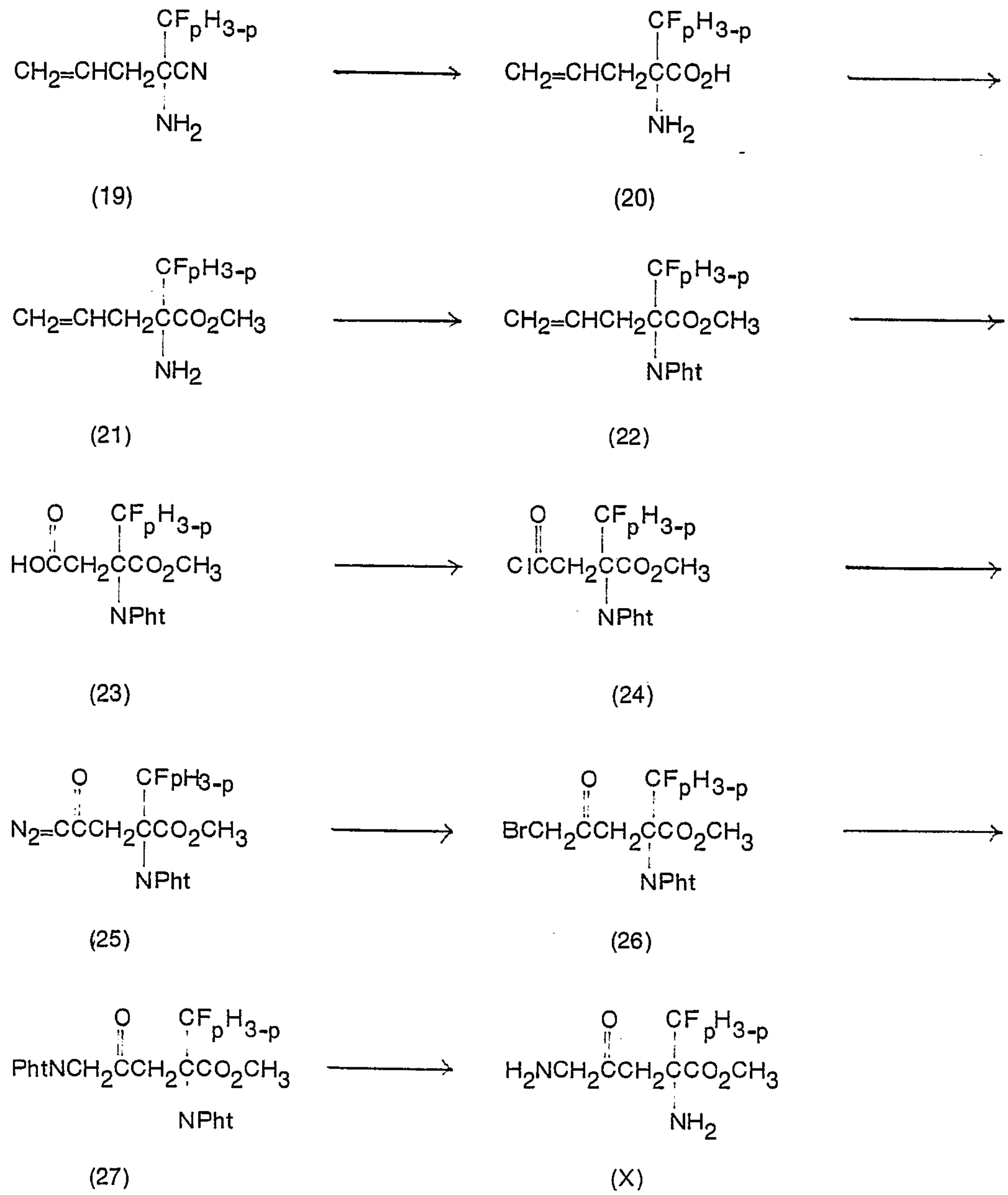

(19) (20) (21) (22) (23) (24) (25) (26) (27) (X)

In Scheme D, PhtN, NPht, and *p* have the meanings as employed in Scheme B.

In the first step of Scheme D, the fluorinated amino-nitrile (19) undergoes acid hydrolysis to give the corresponding fluorinated amino-acid (20) which is then esterified to give the amino-acid ester (21). Treatment of the ester (21) with phthaloyldichloride gives the N-protected amino acid ester (22). Reaction of the N-protected amino acid ester (22) with potassium permanganate oxidizes the double bond and provides the N-protected amino diacid half ester (23) which is converted to the corresponding acid chloride (24) using thionyl chloride. Reaction of the acid chloride (24) with diazomethane yields the corresponding diazoketone (25), which upon reaction with hydrogen bromide, gives the corresponding N-protected $\delta$-bromo $\gamma$-oxo amino acid ester (26). Reaction of this intermediate with potassium phthalimide forms the N,N'-diprotected $\gamma$-oxo ornithine ester (27). Treatment of the ester (27) with hydrochloric acid at 100°C removes the amino protecting groups and hydrolyzes the ester function to give the desired fluorinated $\gamma$-oxo ornithine derivative (X).

As will be apparent to those skilled in the art, the first two steps in the reaction sequence of Scheme D can be omitted. During the subsequent reaction sequence, the nitrile function remains intact up to the final step where it (rather than the ester function) is hydrolyzed to generate the carboxylic acid function.

The fluorinated $\gamma$-keto ornithine derivatives of Formula X can be converted to the fluorinated histidine derivative of Formula XI in two steps. The first step entails reaction with potassium thiocyanate to form the imidizole (28)

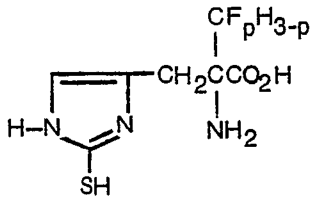

(28)

wherein *p* has the meaning hereinbefore defined.

In the second step, the imidazole (28) is treated with ferric chloride, which removes the —SH group to afford the desired product (XI).

The products of Formula VII wherein $R_7$ is hydrogen, in general, can be prepared in known manner by direct acid hydrolysis of the amino-nitrile intermediate of Formula I wherein R is the group $R_3CH_2$—. The amino-nitrile intermediate can be hydrolyzed in one step or stepwise to give the corresponding amino acid product. In a one-step process the hydrolysis can be performed using a concentrated acid, such as hydrogen bromide at 80°C to 125°C for about 0.5 to 24 hours, under which conditions hydrolysis of the nitrile function is also accompanied by cleavage of the alkoxy groups present in the phenyl ring. In a step-wise process the amino-nitrile intermediate is treated with a lower alcohol (e.g. methanol or ethanol) saturated with anhydrous hydrogen chloride for 1 to 24 hours, preferably 10 hours, at about 0° to 50°C, preferably 25°C, to give the corresponding amino acid amide, which can then be hydrolyzed to the amino acid by treatment with 50% aqueous sulfuric acid for about 2 to 6 hours at about 60° to 100°C, preferably 95°C. Excess sulfuric acid must be removed. Following formation of the amino acid, the alkoxy group can be removed by treatment with concentrated hydrobromic acid at elevated temperatures (such as hereinbefore described) to give the final product.

It will be recognized by those skilled in the art, that when it is desired to prepare a product of Formula VII in which the phenyl moiety contains an alkoxy group *and* a hydroxy group, an intermediate of Formula I, wherein R is:

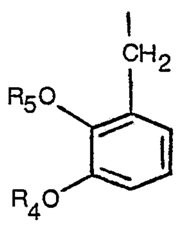

,

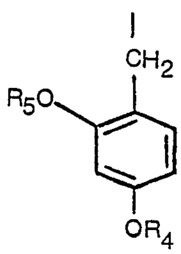

, or

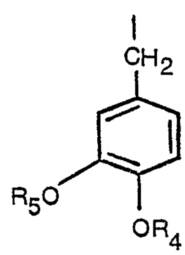

wherein $R_4$ is $C_1$—$C_8$ alkyl and $R_5$ is benzyl or trimethylsilyl, must be employed since the benzyloxy or trimethylsilyloxy group ($OR_5$) can be cleaved preferentially in the presence of the alkoxy group ($OR_4$). Using this method the nitrile group is hydrolyzed and the benzyl or trimethyl-silyl group is cleaved under conditions which do not affect the alkoxy group ($OR_4$). Conditions for carrying out such selective transformations are known in the art.

When it is desired to prepare a compound of Formula VII wherein $R_7$ is $C_1$—$C_8$ alkyl, the corresponding carboxylic acid ($R_7$ is hydrogen) can be esterified by conventional methods, such as by treating the acid with an alkanol saturated with hydrogen chloride. The methyl esters are preferred.

The fluorinated ornithine derivatives of Formula VI, the fluorinated lysine derivatives of Formula VIII, the fluorinated *trans*-dehydroornithine derivatives of Formula V, the fluorinated $\beta$-oxo ornithine derivatives of Formula XIII, the fluorinated $\beta$-methylene ornithine derivatives of Formula XII, the fluorinated $\gamma$-oxo ornithine derivatives of Formula X, the fluorinated $\gamma$-methylene ornithine derivatives of Formula IX, and the fluorinated $\delta$-alkyl ornithine derivatives of Formula $XIV_a$ and $XIV_b$ produce irreversible inhibition of ornithine decarboxylase enzyme (ODC) *in vivo*, and can be used systemically in general to decrease putrescine, spermidine, and/or spermine concentrations in cells undergoing rapid growth or proliferation. Because putrescine, spermidine, and/or spermine concentrations are decreased, the administration of a compound of Formula V, VI, VIII, IX, X, XII, XIII, $XIV_a$ or $XIV_b$ provides a method for controlling undesirable cell growth or, proliferation in mammals. The compounds are useful phamacological agents for treating those diseases or conditions that are known in the art to be characterized by rapid cell growth or proliferation associated with high ODC activity. In particular, the compounds are useful systemically for controlling the growth of tumor tissues in mammals and for

controlling the growth of pathogenic parasitic protozoa in infected domestic animals and humans. The compounds can also be employed to study the presence and physiological function of ODC inhibition in biological system and its relationship to pathological processes.

The compounds of Formula VII produce irreversible inhibition of AADC enzyme *in vivo* and are, in particular, useful for the treatment of Parkinson's syndrome when administered in combination with exogenous dopa, in particular L-dopa. The co-administration of a compound of Formula VII with L-dopa potentiates the effect of L-dopa and thereby provides effective therapy of Parkinsonism using substantially lower doses of L-dopa resulting in a decrease in side effects. The compounds of Formula VII potentiate L-dopa by preventing the peripheral decarboxylation to dopamine, thereby increasing the circulating amount of exogenous L-dopa available for absorption into the brain and subsequent conversion there to dopamine.

The compounds of Formula XI produce irreversible inhibition of histidine decarboxylase *in vivo*, and can be used in general to block histamine biosynthesis in mammals. The compounds of Formula XI are useful pharmacological agents for decreasing the concentrations of histamine in mammals and for the treatment of diseases or conditions characterized by excessive histamine levels. For example, the compounds can be employed for treating allergic conditions and gastric hypersecretory states. The compounds of Formula XI are described in Belgian Patent No. 869,322 granted August 14, 1978.

Preferred examples of fluorinated amino-nitriles of Formula I that can be made by the process of this invention are:

2-fluoromethyl-2-amino-5-methoxy-pentanenitrile,
2-difluoromethyl-2-amino-5-methoxy-pentanenitrile,
2-fluoromethyl-2-amino-5-benzyloxy-pentanenitrile,
2-difluoromethyl-2-amino-5-benzyloxy-pentanenitrile,
2-fluoromethyl-2-amino-3-(E)-pentenenitrile,
2-difluoromethyl-2-amino-3-(E)-pentenenitrile,
2-fluoromethyl-2-amino-5-methoxy-3-(E)-pentenenitrile,
2-difluoromethyl-2-amino-5-methoxy-3-(E)-pentenenitrile,
2-fluoromethyl-2-amino-6-methoxyhexanenitrile,
2-difluoromethyl-2-amino-6-methoxyhexanenitrile,
2-fluoromethyl-2-amino-6-benzyloxyhexanenitrile,
2-difluoromethyl-2-amino-6-benzyloxyhexanenitrile,
2-fluoromethyl-2-amino-4-methyl-4-pentenenitrile,
2-difluoromethyl-2-amino-4-methyl-4-pentenenitrile,
2-fluoromethyl-2-amino-4-methoxymethyl-4-pentenenitrile,
2-difluoromethyl-2-amino-4-methoxymethyl-4-pentenenitrile,
2-fluoromethyl-2-amino-4-pentenenitrile,
2-difluoromethyl-2-amino-4-pentenenitrile,
2-fluoromethyl-2-amino-5-hexenenitrile,
2-difluoromethyl-2-amino-5-hexenenitrile,
2-fluoromethyl-2-amino-3-methoxyethyl-3-butenenitrile,
2-difluoromethyl-2-amino-3-methoxyethyl-3-butenenitrile,
2-fluoromethyl-2-amino-3-benzyloxyethyl-3-butenenitrile,
2-difluoromethyl-2-amino-3-benzyloxyethyl-3-butenenitrile,
2-amino-2-fluoromethyl-3-(3,4-dimethoxyphenyl)propionitrile,
2-amino-2-difluoromethyl-3-(3,4-dimethoxyphenyl)propionitrile,
2-amino-2-fluoromethyl-3-(2,3-dimethoxyphenyl)propionitrile,
2-amino-2-fluoromethyl-3-(2,5-dimethoxyphenyl)propionitrile,
2-amino-2-fluoromethyl-3-(4-methoxyphenyl)propionitrile,
2-amino-2-fluoromethyl-3-(3-methoxyphenyl)propionitrile,
2-amino-2-fluoromethyl-3-(2-methoxyphenyl)propionitrile,

It will be appreciated that the process of the invention is of more or less general application to the preparation of 2-fluorinated methyl aminoacetonitriles and is not restricted to the preparation of the preferred benzyl and alkenyl compounds discussed above.

The invention is illustrated in the following non-limiting Examples of the preparation of intermediates of Formula I and products derived therefrom. All NMR measurements are given on the delta scale (i.e. tetramethylsilane = 0).

Example 1

*2-Fluoromethyl-2,5-diamino-3-(E)-pentene-1-oic acid, monohydrochloride (i.e. α-fluoromethyl-β-trans-dehydroornithine*

A) 2-Fluoromethyl-2-amino-3-pentenenitrile (cis/trans)

Under an atmosphere of nitrogen, propenyl magnesium bromide is prepared from magnesium

turnings (9.8 g, 400 mmoles), freshly distilled 1-bromo-1-propene (24.2 g, 200 mmoles) and 200 mL of dry tetrahydrofuran. After removing the Grignard solution from the excess of magnesium and cooling to −40°C, fluoroacetonitrile (11.8 g, 200 mmoles) in tetrahydrofuran (70 mL) is added during 15 mins. The reaction mixture is then poured into a solution of sodium cyanide (40 g) and ammonium chloride (59 g) in water (400 mL) and kept 1 hour at room temperature. After saturation with sodium chloride, the tetrahydrofuran layer is separated and evaporated under reduced pressure. The residue is dissolved in diethyl ether, washed with water, dried with sodium sulfate and evaporated to give crude 2-fluoromethyl-2-amino-3-pentenenitrile (1:1 cis/trans mixture, 21.5 g) as a dark coloured oil which is used for the next step without further purification.

NMR ($CDCl_3$):

*cis:* *) 1.97 (3H, d of narrow d, J=7Hz, $J_{allyl}\simeq$2Hz); 2.20 (—$NH_2$, broad s), 4.32 (2H, d of AB, $J_{AB}$=8.5Hz, $J_{H-F}$=46Hz), 5.12 (1H, center of A part of $ABX_3$, $J_{AB}$=12Hz, additional allylic coupling), 5.82 (1H, center of B part of $ABX_3$, 2q, $J_{AB}$=12Hz, $J_{BX}$=7Hz).

*trans:* *) 1.77 (3H, d of narrow d, J=7Hz, $J_{allyl}\simeq$1Hz), 2.20 (—$NH_2$, broad s), 4.25 (2H, d, $J_{HF}$=46Hz), 5.27 (1H, center of A part of $ABX_3$, $J_{AB}$=15Hz, additional allylic coupling), 6.13 (1H, center of B part of $ABX_3$, 2q, $J_{AB}$=15Hz, $J_{BX}$=7Hz).

* Assignment of signals is possible by partial separation of the isomers by liquid-liquid partition (water/diethyl ether).

B) 2-Fluoromethyl-2-trifluoroacetamido-3-pentenenitrile

2-Fluoromethyl-2-amino-3-pentenenitrile (21.5 g, 168 mmoles) prepared as in Step A above dissolved in methylene chloride (300 mL) is cooled to −30°C and treated with trifluoroacetic anhydride (34.8 g, 166 mmoles). The mixture is allowed to warm up to room temperature overnight and the solvent is removed under reduced pressure. Dissolving the residue in ethyl acetate, washing with water, drying with sodium sulfate and evaporation gives a dark oil (35 g). TLC (ethyl acetate/petroleum ether 40:60) indicates two major spots with Rf 0.65 anmd 0.60 corresponding respectively to the cis and trans isomers of 2-fluoromethyl-2-trifluoroacetamido-3-pentenenitrile.

Rapid chromatography and collection of the fractions corresponding to these Rf values gave 2-fluoromethyl-2-trifluoroacetamido-3-pentenenitrile (1:1 cis/trans mixture, 23.5 g) as a slightly yellow oil.

NMR ($CDCl_3$):

*cis:*** 1.93 (3H, d, J—7Hz, small allylic coupling), 4.68 (2H, d, $J_{H-F}$=46Hz), 5.37 (1H, center of A part of $ABX_3$, $J_{AB}$=12Hz, additional allylic coupling), 6.00 (1H, center of B part of $ABX_3$, 2q, $J_{AB}$=12Hz, $J_{BX}$=7Hz), 7.6 (NH, broad s).

*trans:*** 1.82 (3H, d, J=7Hz, small allylic coupling), 4.62 (2H, d, $J_{H-F}$=46Hz), 5.40 (1H, center of A part of $ABX_3$, $J_{AB}$=15Hz, additional allylic coupling), 6.27 (1H, center of B part of $ABX_3$, 2q, $J_{AB}$=15Hz, $J_{BX}$=7Hz), 7.6 (NH, broad s).

** A small amount of cis/trans mixture is partially separated into the isomers by chromatography.

C) 2-Fluoromethyl-2-trifluoroacetamido-5-bromo-3-(E)-pentenenitrile

A mixture of 2-fluoromethyl-2-trifluoroacetamido-3-pentenenitrile, (23.5 g, 105 mmoles) prepared as in Step B above, N-bromosuccinimide (19 g, 107 mmoles), carbon tetrachloride (160 mL), and benzoylperoxide is irradiated and heated under reflux by means of a 325 W lamp for 30 mins. During this time, succinimide separates together with an oil which are both collected by filtration on a fritted glass. Dissolving in chloroform, removal of succinimide by filtration and evaporation gives crude 2-fluoromethyl-2-trifluoroacetamido-5-bromo-3-(E)-pentenenitrile (31 g) as a brown oil which is used for the next step without further purification.

NMR ($CDCl_3$):

2.75 (succinimide), 3.97 (2H, d, J=7Hz), 4.68 (2H, d, $J_{H-F}$=46Hz), 5.73 (1H center of A part of $ABX_2$, $J_{AB}$=15Hz), 6.43 (1H, center of B part of $ABX_2$, 2t, $J_{AB}$=15Hz, $J_{BX}$=7Hz), 7.75 (NH, broad s).

D) 2-Fluoromethyl-2-trifluoroacetamido-5-hexamethylene tetrammonium-3-(E)-pentenenitrile bromide

To a solution of 2-fluoromethyl-2-trifluoroacetamido-5-bromo-3-(E)-pentenenitrile (15.0 g, 49.5 mmoles) prepared as in Step C above in chloroform (40 mL) is added hexamethylenetetramine (6.93 g, 49.5 mmoles) in 80 mL of chloroform. A brown oil separates which solidifies on standing overnight. The crude product (14.7 g) is recrystallized by dissolving in hot methanol and addition of twice the volume of chloroform to give pure 2-fluoromethyl-2-trifluoroacetamido-5-hexamethylenetetrammonium-3-(E)-pentenenitrile bromide (11.3 g) as heavy colourless crystals containing 1 mole of chloroform; mp 147°C.

NMR ($CD_3OD$):

3.50 (2H, broad d, J=6—7Hz), 4.43 (6H, s), 4.63 (2H, d, $J_{H-F}$=76Hz), 4.95 (6H, s), 6.07 (2H, m), 7.58 (1H, $CHCl_3$, s)

Analysis for $C_{15}H_{20}BrCl_3F_4N_6O$

Calculated: C, 32.02; H, 3.58; N, 14.94;
Found: C, 32.00; H, 3.51; N, 15.30.

E) 2-Fluoromethyl-2,5-diamino-3-(E)-penten-1-oic acid

2-Fluoromethyl-2-trifluoroacetamido-5-hexamethylene-tetrammonium-3-(E)-pentenenitrile bromide (5.62 g, 10 mmoles) prepared as in Step D above is dissolved in conc. hydrochloric acid (100 mL) and evaporated to dryness at 35°C under reduced pressure. This operation is repeated twice more. The residue is heated with conc. hydrochloric acid (100 mL) at 100°C overnight (16 hours) whilst a slow stream of nitrogen is passed through the solution. After evaporating the dark coloured reaction mixture under reduced pressure and drying for several hours with an oil pump, the residue is dissolved in dry ethanol (50 mL), ammonium chloride is removed by filtration and propylene oxide (1.8 g) is added to precipitate the crude monohydrochloride. After standing at 5°C overnight, a brown, hygroscopic precipitate is collected on a fritted glass, washed with a small amount of dry ethanol, dissolved in water and treated with charcoal at 40°C—50°C for 2 hours. Evaporation of the colourless filtrate gives a white solid which is recrystallized to give pure 2-fluoromethyl-2,5-diamino-3-(E)-penten-1-oic-acid, monohydrochloride (730 mg,) as colourless crystals, mp 176°C (dec).

NMR ($D_2O$/DCL):

3.83 (2H, broad d), 4.87 (1H centre of A part of ABX, $J_{AB}$=11Hz, $J_{AX}$=$J_{H-F}$=47Hz), 5.23 (1H, center of B part of ABX, JAB=11Hz, $J_{BX}$=H$J_{H-F}$=45Hz), 6.18 (2H, m).

Analysis for $C_6H_{12}ClFN_2O_2$

Calculated: C, 36.28; H, 6.09; N, 14.10
Found: C, 36.10; H, 5.88; N, 13.85

Example 2

*5-Fluoromethyl-2,5-diaminovaleric acid (α-Fluoromethyl-ornithine)*

A. 2-Fluoromethyl-2-amino-5-methoxy-valeronitrile

Under an atmosphere of nitrogen, 3-methoxypropyl magnesium chloride is prepared from 3-methoxy-1-chloropropane (5.43 g, 50 mmol, prepared according to Haworth and Perkin, Chem. Zentralblatt II 1271 (1912) and magnesium turnings (1.22 g, 50 mmol) in dry THF (50 ml). The mixture is heated under reflux for 3 hours, then cooled to −30°C and a solution of fluoroacetonitrile (2.95 g, 50 mmol) in THF (30 ml) is added during 20 minutes. After keeping the mixture at −30°C for 1/2 hour more, a solution of sodium cyanide (4.9 g, 100 mmol) and ammonium chloride (8.09 g, 150 mmol) in water (100 mL), previously cooled to 0°C, is added and the mixture is stirred for 3/4 hours at room temperature. After saturating with sodium chloride, the THF layer is separated and the aqueous phase is extracted twice with ether. After drying ($Na_2SO_4$), the combined organic extracts are evaporated to give 2-fluoromethyl-2-amino-5-methoxyvaleronitrile (4.0 g) as a brown oil.

NMR ($CDCl_3$) $\delta$:

1.77 (4H, m), 2.10 (broad s, $NH_2$), 3.30 (3H, s), 3.40 (2H, t), 4.32 (2H, ABX, $J_{AB}$=9Hz, $J_{AX}$=$J_{BX}$=$J_{H-F}$=47Hz.

B) 2-Fluoromethyl-2-phthalimido-5-methoxy-valeronitrile

To a solution of 2-fluoromethyl-2-amino-5-methoxy-valeronitrile (1.62 g, 10 mmol) and triethylamine (2.02 g, 20 mmol) in methylene chloride (30 ml), cooled to −20°C, is added phthaloyldichloride (2.03 g, 10 mmol) in methylene chloride (10 mL). The mixture is allowed to warm up to room temperature overnight. After washing with water, 1N HCl, water again, and drying ($Na_2SO_4$), the solvent is removed under reduced pressure to give 2.4 g of crude material. This is purified by chromatography on silica (ethyl acetate/petroleum ether 3:7).

NMR ($CDCl_3$) $\delta$:

2.15 (4H, m), 3.23 (3H, s), 3.40 (2H, t, J=6Hz), 5.02 (2H, ABX, $J_{AB}$=9Hz, $J_{AX}$=$J_{BX}$=$J_{H-F}$=46Hz), 7.77 (4H, s).

C) 2-Fluoromethyl-2-phthalimido-5-iodo-valeronitrile

2-Fluoromethyl-2-phthalimido-5-methoxy-valeronitrile (1.20 g, 4.14 mmol), trimethylsilyl iodide

(3.2 g, 16 mmol) and chloroform (15 ml) are heated to 60°C under nitrogen for 48 hours. After removal of the solvent, the residue is dissolved in chloroform, washed with water, sodium thiosulfate solution and water again, dried and evaporated to give the crude product as an oil (1.2 g). This is purified by chromatography on silica (ethyl acetate/petroleum ether 1:3) to give pure 2-fluoromethyl-2-phthalimido-5-iodo-valeronitrile.

NMR ($CDCl_3$) $\delta$:
2.0 (4H, m), 3.10 (2H, t), 4.90 (2H, ABX, $J_{AB}$=9Hz, $J_{AX}$=$J_{BX}$=$J_{H-F}$=46Hz), 7.70 (4H, s).

D) 2-Fluoromethyl-2,5-diphthalimido-valeronitrile

2-Fluoromethyl-2-phthalimido-5-iodo-valeronitrile (1.20 g, 3.11 mmol) and potassium phthalimide (0.75 g, 4 mmol) are heated in dimethylformamide (25 ml) to 80°C for 2 hours. After standing overnight at room temperature, the DMF is removed by vacuum distillation and the residue is dissolved in chloroform and washed with 1N KOH and water. After drying ($Na_2SO_4$), evaporation gives 2-fluoromethyl-2,5-diphthalimido-valeronitrile as a solid.

NMR ($CDCl_3$) $\delta$:
2.17 (4H, m), 3.73 (2H, t), 4.93 (2H, ABX, $J_{AB}$=9Hz, $J_{AX}$=$J_{BX}$=$J_{H-F}$=46Hz), 7.73 (8H, broadened s).

E) 2-Fluoromethyl-2,5-diaminovaleric acid

2-Fluoromethyl-2,5-diphthalimido valeronitrile (1.21 g, 3 mmol) is refluxed with conc. hydrochloric acid (20 ml) for 4 days. After standing at room temperature for several hours, phthalic acid is removed by filtration, the filtrate is evaporated, the residue dissolved in 2N HCl (20 ml) and carefully extracted with ether (5 × 10 ml). After evaporation, the residue is dried carefully under vacuum (oil pump) overnight. It is dissolved in dry ethanol (7 ml) and, after filtration, propylene oxide (0.3 g, 5 mmol) in ethanol (1 ml) is added to precipitate the monohydrochloride. This is collected after standing overnight at room temperature and recrystallized from water/ethanol to give pure 2-fluoromethyl-2,5-diamino-valeric acid, monohydrochloride, mp 260°C (dec); TLC (EtOH/$NH_4OH$ 80/20): 0.18.

NMR ($D_2O$) $\delta$:
1.93 (4H, m), 3.10 (2H, broad t), 4.83 (2H, ABX, $J_{AB}$=10Hz, $J_{AX}$=$J_{BX}$=$J_{H-F}$=46Hz).

Example 3

*2-Amino-2-fluoromethyl-3-(2,3-dihydroxyphenyl) propionic acid*

A) 2,3-Dimethoxybenzylchloride

Thionyl chloride (288 g) is added over 1.5 hour to a stirred solution of 2,3-dimethoxybenzyl alcohol (400 g) and 2,6-lutidine in methylene chloride (2 l). The reaction mixture is then stirred for 30 minutes after which it is washed with 2 N hydrochloric acid (7 × 1 l) and then with water. The organic layer is separated, dried ($MgSO_4$) and evaporated to give a residue, which upon distillation yields 2,3-dimethoxybenzyl chloride (375 g), b.p. 145—150°C at 20—25 nm (waterpump vacuum). The material solidifies on standing.

B) 2-Amino-2-fluoromethyl-3-(2,3-dimethoxyphenyl)propionitrile

Ethyl bromide (1 ml) is added to magnesium turnings (48 g) and magnesium powder (48 g) covered with tetrahydrofuran (THF) (800 ml). 2,3-Dimethoxybenzyl chloride (336 g) in THF (2000 ml) is then added over 2.5 hours while the reaction mixture is maintained at 20°C by means of a water bath. After the addition, the mixture is stirred for one additional hour and excess magnesium is removed by decantation. The Grignard reagent thus obtained is cooled to −35°C and fluoroacetonitrile (105 g) in THF (600 ml) is added at −40°C to −30°C. Cooling is continued for one hour. The reaction mixture is poured into a solution of sodium cyanide (175 g) and ammonium chloride (275 g) in water (4 l) at 10°C. After the mixture is stirred for 30 minutes, the organic phase is separated. The water phase is saturated with sodium chloride (250 g) and is washed with ether (3 × 1 l). The combined organic phases are dried ($MgSO_4$), filtered, and evaporated to dryness. The residue, dissolved in ether (2 l), is extracted with 10% hydrochloric acid (4 × 250 ml) and the acid phases are combined, extracted with diethyl ether, and made basic with conc. ammonium hydroxide. The oil, which separates, is taken up into diethyl ether (1 l), and the solution is dried ($MgSO_4$), filtered, and evaporated to give the propionitrile product (110 g) as a brown oil.

C) 2-Amino-2-fluoromethyl-3-(2,3-dihydroxyphenyl)propionic acid

2-Amino-2-fluoromethyl-2-(2,3-dimethoxyphenyl)propionitrile (100 g) and 48% hydrobromic acid (500 ml) are heated under reflux overnight. The hot solution is treated with charcoal (5 g), filtered, and evaporated to dryness over water-pump vacuum. The residue is treated with water (250 ml) and the mixture taken to dryness. This procedure is repeated. The residue is taken up in hot isopropanol

(500 ml). After filtration of the insoluble ammonium bromide, the solution is concentrated (to about 400 ml). Trimethylamine is then added to adjust the pH to 5, and diethylether (1 l) is added. A solid which separates is collected and washed with chloroform 4 x 250 ml). The remaining solid is dried and taken up in boiling water (1 l). Charcoal is added. After removal of the charcoal by filtration, the mixture is concentrated (to about 400 ml) and cooled. The light brown solid which separates is collected and washed with cold isopropanol and diethylether. Recrystallization three times from water, gives the title product (24 g), m.p. 224°C (dec).

## Example 4

*2-Amino-2-fluoromethyl-3-(2,5-dihydroxyphenyl)propionic acid*

A) Fluoroacetonitrile

A stirred mixture of phosphorus pentoxide (200 g), fluoroacetamide (150 g) and hexamethylphosphoramide (500 ml), in a 2 l reactor fitted with a simple distillation head, a distillation condenser and a receiver cooled in a mixture of solid carbon dioxide/acetone, is slowly heated to 50°C under a pressure of 90 mm Hg. At this temperature a vigorous reaction commences and fluoroacetonitrile begins to distil at 38°C.

After the vigorous reaction has subsided (about 3 minutes) the temperature of the oil bath is slowly raised to 140°C and the remaining fluoroacetonitrile is collected at 38—50°C. The fluoroacetonitrile (111 g) is obtained as a colorless liquid. An NMR analysis shows that it contains traces of hexamethyl phosphoramide which can be removed, if desired, by a redistillation, but its presence is not detrimental for the subsequent Grignard reaction.

B) 2,5-Dimethoxybenzyl chloride

Thionyl chloride (108 g) is slowly added during 1 hour to a stirred mixture of 2,5-dimethoxybenzyl alcohol (150 g), 2,4,6-collidine (108 g), and methylene chloride (750 ml) at room temperature. The reaction is slightly exothermic. After stirring for another 30 minutes, the mixture is washed with hydrochloric acid (2 N) and water and then dried ($MgSO_4$). Evaporation gives a residue of 2,5-dimethoxybenzyl chloride which crystallizes (127 g).

Analysis for $C_9H_{11}Cl$:

| | |
|---|---|
| Calculated: | C, 57.92; H, 5.94; % |
| Found: | C, 57.93; H, 5.94; % |

C) 2-Amino-2-fluoromethyl-3(2,5-dimethoxyphenyl)propionitrile

Ethyl bromide (2 ml) is added to magnesium turnings (25 g, 1 mol) covered with anhydrous tetrahydrofuran at room temperature under nitrogen. When the reaction has subsided, 2,5-dimethoxybenzyl chloride (102.5 g, 0.55 mol) in anhydrous tetrahydrofuran (200 ml) is slowly added at such a rate to cause a gentle reflux. After stirring for a further hour, the Grignard solution is decanted from the excess magnesium (11.7 g), cooled to −35°C, and treated with a solution of fluoroacetonitrile (33.6 g, 0.59 mol) in anhydrous tetrahydrofuran (200 ml) by dropwise addition, a nitrogen atmosphere being maintained throughout. The mixture is stirred for a further hour at −35°C and then poured into a stirred solution of sodium cyanide (55 g) and ammonium chloride (88 g) in water (1 l) at 10°C. The mixture is stirred for 30 minutes. The organic phase is then separated. The aqueous phase is saturated with sodium chloride and extracted with ether (2 x). The combined organic phases are charcoaled, dried ($MgSO_4$), filtered, and evaporated to give an oil (112 g). A solution of the oil in anhydrous diethylether is treated with ethereal hydrogen chloride and the precipitated oil is crystallized by trituration. The solid is filtered and washed well with ether to give crude 2-amino-2-fluoromethyl-3(2,5-dimethoxyphenyl)-propionitrile as the hydrochloride (80 g).

D) 2-Amino-2-fluoromethyl-3(2,5-dimethoxyphenyl)propionamide

The amino nitrile prepared in Step C (80 g) is dissolved in the minimum of methanol and treated with an equal volume of methanol saturated with hydrogen chloride at 0°C. The mixture is allowed to stand in a refrigerator overnight and then evaporated. The residue is crystallized by trituration under ether to give 2-amino-2-fluoromethyl-3(2,5-dimethoxyphenyl)propionamide as the hydrochloride (68 g).

E) 2-Amino-2-fluoromethyl-3(2,5-dihydroxyphenyl)propionic acid

A mixture of the amide prepared in Step D (68 g) in 47% hydrobromic acid (250 ml) is refluxed for 1 night under nitrogen. The hot solution is treated with charcoal, filtered, and evaporated to dryness. Dioxane is added to the residue and evaporated to remove the last traces of water. Dioxane treatment is repeated once. A solution of the residue in isopropanol is filtered to remove ammonium bromide and concentrated to about 250 ml. The pH of the solution is adjusted to 5 by the addition of triethylamine in isopropanol to begin crystallization. Anhydrous ether (approx. 1 l) is added, the mixture is filtered, and

the solid so obtained is washed with chloroform to remove triethylamine hydrobromide.

The solid is dissolved in water (1 l at 100°) and the solution is charcoaled and evaporated to about 500 ml to give crystals of 2-amino-2-fluoromethyl-3-(2,5-dihydroxyphenyl)propionic acid as the dihydrate (21 g). A further 10 g is obtained by concentration of the mother liquors, m.p. 212°C.

Example 5

*2-Amino-2-fluoromethyl-3-(2-hydroxy-3-methylphenyl)propionic acid*

A) 2-Amino-2-fluoromethyl-3-(2-methoxy-3-methyl)phenyl propionitrile

All manipulations are carried out in an atmosphere of nitrogen.

A solution of ethylene dichloride (10.59 g, 0.107 mol) in anhydrous tetrahydrofuran (THF) (150 ml) is slowly added to a stirred mixture of magnesium turnings (2.59, 0.107 mol) and anhydrous THF (50 ml). The vigorous exothermic reaction is controlled by cooling with ice-cold water. The reaction is finally warmed at 30°C to dissolve all the magnesium and the solution so obtained is added to a freshly prepared solution of sodium (4.9 g, 0.214 mol) in a mixture of naphthalene (28.2 g, 0.22 mol) and anhydrous tetrahydrofuran (200 ml). An intermediate fine black suspension of magnesium is formed.

Magnesium turnings (2.5 g, 0.107 mol) are added to the above suspension to maintain an excess of magnesium, and the stirred mixture cooled to —20°C. A solution of 2-methoxy-3-methylbenzyl chloride (18.3 g, 0.107 mol) in THF (50 ml) is added during an hour, the black color of the mixture disappearing at the end of the addition. The solution is decanted from the excess magnesium and treated with a solution of fluoroacetonitrile (6.3 g, 0.107 mol) in anhydrous tetrahydrofuran (50 ml) at —20°C by dropwise addition during 30 minutes. The mixture is stirred for a further hour at —20°C and poured into a stirred aqueous solution of a mixture of sodium cyanide (10.49 g, 0.214 mol) and ammonium chloride (17.2 g, 0.32 mol).

After half an hour, the organic layer is separated using diethyl ether, dried, and concentrated to 1/3 volume. Ethereal hydrogen chloride is added and the mixture is allowed to crystallize. The crystals of 2-amino-2-fluoromethyl-3-(2-methoxy-3-methylphenyl)propionitrile, hydrochloride (m.p. 128°C) are filtered off and recrystallized from acetonitrile (8 g).

B) 2-Amino-2-fluoromethyl-3-(2-methoxy-3-methylphenyl)propionamide

A solution of 2-amino-2-fluoromethyl-3-(2-methoxy-3-methylphenyl)propionitrile hydrochloride (5.2 g) in methanol saturated with hydrogen chloride (100 ml) is allowed to stand for 64 hours in a refrigerator. The methanol is evaporated, the residue is dissolved in water, and the solution basified by the addition of aqueous potassium carbonate. The organic material (4.8 g) is isolated by extraction with dichloromethane. The syrup is purified first by column chromatography using silica and a mixture of chloroform 93% and acetone 7% eluant, and finally by crystallization from benzene to give crystals of 2-amino-2-fluoromethyl-3(2-methoxy-3-methylphenyl)propionamide, m.p. 118°C (3.5 g).

C) 2-Amino-2-fluoromethyl-1-3-(3-hydroxy-3-methylphenyl)propionic acid

A solution of 2-amino-2-fluoromethyl-3-(2-methoxy-3-methylphenyl)propionamide (1.5 g) in 48% hydrobromic acid is refluxed overnight, the acid evaporated, and the residue dissolved in isopropanol. After removing the ammonium bromide by filtration, the pH of the solution is adjusted to 5 by the addition of triethylamine. The crystals which separate are filtered, washed well with chloroform, and dissolved in water. The aqueous solution is evaporated and the residue crystallized from ethanol to give 2-amino-2-fluoromethyl-3-(2-hydroxy-3-methylphenyl)propionic acid, as the semi-hydrate, m.p. 168°C.

Example 6

*2-Amino-2-fluoromethyl-3-(3-hydroxy-4-methoxyphenyl)propionic acid*

A) 2-Amino-2-fluoromethyl-3-(3-benzyloxy-4-methoxyphenyl)propionitrile

Under an atmosphere of nitrogen, 3-benzyloxy-4-methoxybenzyl magnesium chloride is prepared from 3-benzyloxy-4-methoxybenzylchloride (C. Schöpf and L. Winterhalder, Ann. *544*, 62 (1940) (22.0 ng, 84 mmol) and magnesium turnings (4.1 g) in tetrahydrofuran (THF) (100 ml) at room temperature (about 2 hours). After cooling to —30°C, a solution of fluoroacetonitrile (4.94 g) in THF (40 ml) is added dropwise, maintaining the temperature between —30°C and —40°C. Stirring is continued at this temperature for another 30 minutes, then the mixture is poured into a solution of sodium cyanide (12.3 g) and ammonium chloride (17.9 g) in water (150 ml), previously cooled with ice. After stirring for 30 minutes, the mixture is saturated with sodium chloride whereupon the phases separate. After extracting the aqueous phase two more times with diethyl ether, the combined THF and ether phases are dried ($Na_2SO_4$) and evaporated to give the crude aminonitrile (25.9 g) as a brown oil. The oil is dissolved in ether and treated with HCl gas to prepare the hydrochloride as an oil. This oil is recrystallized twice from ethanol/ether to give 2-fluoromethyl-2-amino-3-(3′-benzyloxy-4′-methoxyphenyl)propionitrile as the hydrochloride as a slightly coloured solid;

NMR ($CD_3OC$):

3.47 ppm (3H, s), 4.32 ppm (2H, d, $J_{H-F}$=46Hz), 4.73 ppm (2H, s), 6.60 ppm (3H, m), 6.97 ppm (5H, m).

B) 2-Amino-3-fluoromethyl-3-(3-hydroxy-4-methoxyphenyl)propionic acid

A solution of 2-amino-2-fluoromethyl-3-(3-benzyloxy-4-methoxyphenyl)propionitrile (0.7 g) in absolute methanol saturated with dry hydrogen chloride (50 ml) is heated at reflux for 12 hours. The methanol is evaporated and the residue dissolved in trifluoroacetic acid (10 ml). The mixture is heated at reflux temperature for 2 hours. Water (4 ml) is then added, and reflux continues overnight. The residue obtained upon evaporation under reduced pressure is passed through Amberlite[R] 120 $H^+$ ion exchange resin column. The fractions are pooled and concentrated. The residue is crystallized from water-methanol-ether to afford the desired product.

Example 7

*Methyl 2-amino-2-fluoromethyl-3-(4-hydroxyphenyl)propionate*

A) 2-Amino-2-fluoromethyl-3-(4-methoxyphenyl)propionitrile

Under nitrogen, 4-methoxybenzylmagnesium chloride is prepared by adding 4-methoxybenzyl chloride (160 g) in tetrahydrofuran (THF) (800 ml) to magnesium turnings (50 g) and THF (400 ml) within about 2 hours. The reaction is initiated by the addition of a few drops of methyl iodide, and the reaction flask is cooled by a bath containing water of room temperature. Stirring is continued for an additional 1/2 hour, and the solution is decanted from the excess of magnesium, transferred to a second flask and cooled to —30°C to —40°C. Fluoroacetonitrile (58 g) in THF (250 ml) is added dropwise within 40 minutes, keeping the temperature (internal) between —30°C and —40°C. After the addition, stirring is continued for 10 minutes at the same temperature then the reaction mixture is poured into a stirred solution of sodium cyanide (100 g) and ammonium chloride (100 g) in water (2 l) and stirred for 1 hour at room temperature. Sodium chloride (400 g) is added to separate the phases. The THF layer (upper phase) is removed, and the aqueous layer is extracted with diethyl ether (3 x 1 l). After drying ($Na_2SO_4$) the THF solution and the ethereal extracts are stripped to give 200 g of crude product. Treatment of a solution of the crude nitrile in diethyl ether (3 l) with HCl gas precipitates the hydrochloride which is recrystallized from ethanol/ether (120 g).

B) 2-Amino-2-fluoromethyl(4-hydroxyphenyl)propionic acid

Under nitrogen, 2-amino-2-fluoromethyl-3-(4-methoxyphenyl)propionitrile, hydrochloride (30 g) and conc. hydrobromic acid (500 l) are heated at 90°C for 30 hours. After evaporation, the residue is dissolved in water, and aqueous ammonia is added until pH 5.5 The precipitate is collected, dried and washed several times carefully with acetone. The washed precipitate is dissolved in 2 N hydrochloric acid, treated with charcoal, and reprecipitated by addition of ammonia (pH 5.5). Reprecipitation in the same manner gives 2-amino-2-fluoromethyl-3-(4-hydroxyphenyl)propionic acid (16.6 g), m.p. 239°C (dec.).

Analysis for $C_{10}H_{12}FNO_3$:

| | |
|---|---|
| Calculated: | C, 56.33; H, 5.67; N, 6.57; % |
| Found: | C, 56.34; H, 5.73; N, 6.42; % |

C) Methyl 2-amino-2-fluoromethyl-3-(4-hydroxyphenyl)propionate

With ice cooling, a suspension of 2-amino-2-fluoromethyl-3-(4-hydroxyphenyl)propionic acid (10.0 g) in absolute methanol (400 ml, dried over magnesium) is saturated with HCl gas. After heating at 90°C overnight, the solvent is removed under reduced pressure, and the residue is dried for 3 hours under the vacuum of an oil pump. After dissolving in absolute methanol (400 ml) and saturating with HCl gas, the mixture is heated at 90°C overnight again. After evaporation of the solvent, the residue is dissolved in water and a solution of sodium carbonate is added with stirring until pH approx. 10. The suspension is extracted 3 times with ether; the combined ether extracts are filtered, washed carefully with water, dried ($Na_2SO_4$) and evaporated to give pure methyl-2-amino-2-fluoromethyl-3-(4-hydroxyphenyl)propionate (7.5 g, 70%) as white crystals, m.p. 130°C.

Analysis for $C_{11}H_{14}FNO_3$:

| | |
|---|---|
| Calculated: | C, 58.14; H, 6.21; N, 6.16; % |
| Found: | C, 58.32; H, 6.34; N, 6.18; % |

NMR ($CD_3OD$) $\delta$:

2.77 (2H, AB, $J_{AB}$=14Hz), 3.70 (3H, s), 4.50 (2H, ABX, $J_{AB}$=9Hz, $J_{AX}$=$J_{BX}$=$J_{H-F}$=46Hz), 6.87 (4H, $A_2B_2$ with additional fine splitting, $J_{AB}$=9Hz).

Example 8

*2-Fluoromethyl-2,5-diaminovaleric acid (α-Fluoromethylornithine)*

A) 1-Chloro-3-benzyloxypropane

In a 2 l flask, a mixture of 3-chloropropanol (56.7 g), benzyl bromide (102.6 g) and dry THF (600 ml) is stirred and cooled with ice/salt mixture. Potassium *tert*-butoxide (70 g) is added in portions (5—10 g), maintaining the internal temperature at about 0°C. After the mixture is stirred for 3 more hours at room temperature, the solvent is evaporated. Addition of 1 N HCl (1 l), extraction with ether (1 l), and evaporation of solvent gives crude material (113.6 g), which is distilled under vacuum to give the title compound, (108.4 g), $bp_{0.01}$ 60—80°C.

NMR ($CDCl_3$) δ:
1.97 (2H, q, J=6Hz), 3.50 and 3.55 (4H, 2t, J=7Hz), 4.41 (2H, s), 7.24 (5H, s).

B) 2-Fluoromethyl-2-amino-5-benzyloxyvaleronitrile

To magnesium turnings (30.6 g, 1.26 mol), suspended in dry ether (150 ml) is added methyl iodide (about 0.5 ml) and then a solution of 1-chloro-3-benzyloxypropane (116 g, 0.63 mol) in ether (1.1 l) at such a rate that gentle reflux is maintained. (This procedure is performed under nitrogen). After the mixture is heated for 1 more hour under reflux, titration indicates Grignard formation to be complete. The solution is separated from the excess of magnesium (glass wool), transfered to a 6 l flask and, again under nitrogen, cooled to —40°C. Fluoroacetonitrile (33.48 g, 0.57 mol) in ether (300 ml) is added slowly. The mixture is then kept at —40°C for another 1/2 hour and is poured into a mixture of sodium cyanide (123 g), ammonium chloride (186 g), ice (600 g), and water (650 ml). The mixture is stirred vigorously and allowed to warm up to room temperature during 1 hour. Ether is added, the aqueous phase is saturated with sodium chloride, and the organic layer is separated. The aminonitrile is extracted with 1 N HCl (2 x 750 ml) and re-extracted with ether (2 x 750 ml) after basification with concentrated ammonia. Drying ($Na_2SO_4$) and evaporation gives the title compound as a brown oil (74.8 g).

NMR ($CDCl_3$) δ:
1.38—2.43 (4H, m), 3.45 (2H, m), 4.28 (2H, ABX, $J_{AB}$=9Hz, $J_{AX}$=$J_{BX}$=$J_{H-F}$=47Hz), 4.47 (2H, s), 7.30 (5H, s).

The product can be used for the next step without further purification.

C) 2-Fluoromethyl-2-phthalimido-5-benzyloxy-valeronitrile

To a stirred mixture of 2-fluoromethyl-2-amino-5-benzyloxy-valeronitrile (74.8 g), triethylamine (100 g), and dry dichloromethane (450 ml), cooled in an ice bath, a solution of phthaloyldichloride (58 g) in dichloromethane (300 ml) is added slowly. Stirring is continued at room temperature overnight. The mixture is extracted with 2 N HCl (2 x 750 ml), washed with water (2 x 750 ml), dried carefully ($Na_2SO_4$, and evaporated to give a brown oil which, according to NMR analysis, contains some iso-phthalimide. The oil is dissolved in dry dichloromethane (500 ml), triethylamine (100 g) is added, and the mixture is refluxed for 3 hours. Work-up as described above gives an oil which is purified by flash-chromatography on silica (2 kg, petroleum ether/ethyl acetate 4:1). Crystallisation from ether gives the title compound (49.4 g).

NMR ($CDCl_3$) δ:
1.5—3.0 (4H, m), 3.52 (2H, t), 4.44 (2H, s), 5.03 (2H, ABX, $J_{AB}$=90Hz, $J_{AX}$=$J_{BX}$=$J_{H-F}$=47Hz), 7.30 (5H, s), 7.80 (4H, s).

D) 2-Fluoromethyl-2-phthalimido-5-hydroxy-valeronitrile

To a solution of 2-fluoromethyl-2-phthalimido-5-benzyloxy-valeronitrile (49.4 g) in dry dichloromethane (400 ml) is added TMSI (45 ml, 2.2 equivalents), and the mixture is stirred at room temperature overnight. The solvent is removed under vacuum. The residue is evaporated two times more with dichloromethane and then dissolved in chloroform. After addition of triethylamine (60 ml), the mixture is refluxed for 3 hours. Washing with water, 1 N HCl (2 x), water again, drying, and evaporation gives the title compound as a brown oil (42.8 g).

NMR ($CDCl_3$) δ:
1.0—2.9 (6H + OH, m), 3.65 (2H, t + additional splitting), 5.03 (2H, ABX, $J_{AB}$=9Hz, $J_{AX}$=$J_{BX}$=$J_{H-F}$=47Hz), 7.80 (4H, s).

E) 2-Fluoromethyl-2-phthalimido-5-methane-sulfonyloxy-valeronitrile

A mixture of 2-fluoromethyl-2-phthalimido-5-hydroxy-valeronitrile (42.8 g), dry pyridine (170 ml), and dry dichloromethane (350 ml) is stirred and cooled with ice/salt mixture. Freshly distilled methane-

sulfonylchloride (15.7 g) in dichloromethane (200 ml) is slowly added, and stirring is continued at room temperature overnight. The mixture is then poured onto ice/2 N HCl, and the organic phase is extracted subsequently with 1 N HCl, water, 10% sodium bicarbonate, and water (3 x). Drying ($MgSO_4$), treatment with charcoal (room temperature), and evaporation gives the title compound as a brown oil (42 g).

NMR ($CDCl_3$) $\delta$:
1.1—2.9 (4H, m), 3.03 (3H, s), 4.31 (2H, t + additional splitting), 5.03 (2H, ABX, $J_{AB}$=9Hz, $J_{AX}$=$J_{BX}$=$J_{H-F}$=47Hz), 7.86 (4H, s).

F) 2-Fluoromethyl-2-phthalimido-5-iodo-valeronitrile

A solution of 2-fluoromethyl-2-phthalimido-5-methanesulfonyloxy-valeronitrile (42 g) and sodium iodide (35.7 g) in acetone (600 ml) is stirred and heated under reflux. After about 1/2 hour, the mixture solidifies. Acetone (500 ml) is added, and refluxing and stirring is continued overnight. The salts are removed by filtration and washed several times with acetone. The acetone is evaporated to give a residue which is dissolved in ether (1.5 l). The ether solution is washed subsequently with water (2 ×), sodium sulfite solution, and water again (3 ×). After drying ($MgSO_4$) and evaporating the ether, the title compound is obtained as an oil (34.1 g).

NMR ($CDCl_3$) $\delta$:
1.0—2.9 (4H, m), 3.16 (2H, t), 4.97 (2H, ABX, $J_{AB}$=9Hz, $J_{AX}$=$J_{BX}$=$J_{H-F}$=47Hz), 7.80 (4H, s).

G) Fluoromethyl-2,5-diphthalimido-valeronitrile

A mixture of 2-fluoromethyl-2-phthalimido-5-iodo-valeronitrile (34.1 g), potassium phthalimide (16.6 g), and dry DMF (230 ml) is stirred and heated at 70—80°C for 5 hours. The DMF is removed under reduced pressure, the residue is dissolved in chloroform, and the chloroform solution is washed with water (2 ×), 1 N KOH (2 ×), 1 N HCl, and water again (3 ×). The washed chloroform solution is dried and solvent removed to give an oil which is evaporated twice with chloroform/ether to give a solid. Recrystallization from ether (500 ml) gives the pure title compound (18.5 g).

NMR ($CDCl_3$) $\delta$:
1.5—2.9 (4H, m), 3.78 (2H, t), 5.03 (2H, ABX, $J_{AB}$=9Hz, $J_{AX}$=$J_{BX}$=$J_{H-F}$=47Hz), 7.76 (8H, s).

H) 2-Fluoromethyl-2,5-diaminovaleric acid

2-Fluoromethyl-2,5-diphthalimido-valeronitrile (18.5 g) is heated under reflux with concentrated HCl for 4 days. After the reaction mixture is cooled to room temperature, phthalic acid is removed by filtration, and the filtrate is evaporated to dryness. The residue is dissolved in water, and the water solution is extracted twice with ether and then taken to dryness. Last traces of water and HCl are removed by evaporating the residue twice with isopropanol. The residue is dissolved in ethanol (300 ml), ammonium chloride is removed by filtration, and propylene oxide (9.5 g) is added. The mixture is kept in the refrigerator overnight, and the crude monohydrochloride is collected by filtration. It is dissolved in the minimum amount of water, filtered (Millipore)®, and recrystallized by addition of 5—10 volumes of ethanol to give the title compound as the monohydrochloride, monohydrate (7.6 g).

Analysis for $C_6H_{13}FN_2O_2$, HCl, $H_2O$:

| | |
|---|---|
| Calculated: | C, 32.96; H, 7.38; N, 12.81; % |
| Found: | C, 32.08; H, 7.23; N, 13.13; % |

Example 9

*2-Fluoromethyl-2,6-diaminohexanoic acid (α-Fluoromethyl-lysine)*

A) 4-Benzyloxybutanol

In a 2 l flask, a solution of 1,4-butanediol (45 g, 0.5 mol) and benzyl bromide (85.5 g, 0.5 mol) in dry THF (500 ml) is cooled to 0°C (internal temperature). With vigorous stirring, potassium-tert-butoxide (56 g, 0.5 mol) is added in approx. 5 g portions, maintaining the internal temperature below 10°C. After addition of 1 N HCl (2 l), the mixture is saturated with sodium chloride, extracted with ether (1.5 l), washed with water, dried, and finally evaporated to give an oil (88 g) which is distilled under vacuum to give the pure title compound (63.5 g), $bp_{0.2}$ 113—114°C.

NMR ($CDCl_3$) $\delta$:
1.65 (4H, m), 2.9 (1H, s), 3.53 (4H, m), 4.50 (2H, s), 7.33 (5H, s).

B) 1-Chloro-4-benzyloxybutane

A mixture of 4-benzyloxybutanol (64.8 g, 0.36 mol) in dry pyridine (200 ml) is stirred and heated

to 50—60°C. A solution of thionyl chloride (43 g, 0.36 mol) in pyridine (100 ml) is added slowly, and the mixture is kept at 60°C for one more hour. After cooling to room temperature, the reaction mixture is poured into a mixture of ice and 2 N HCl. The mixture is saturated with sodium chloride, extracted with ether, and washed successively with water, 10% sodium bicarbonate, and water. Evaporation of solvent gives a yellow oil (49.7 g) which is distilled under vacuum to give the pure title compound (47 g), bp $_{0.03-0.05}$ 85—90°C.

NMR ($CDCl_3$) $\delta$:
1.83 (4H, m), 3.88 (4H, m), 4.53 (2H, s), 7.37 (5H, s).

C) 2-Fluoromethyl-2-amino-6-benzyloxy-hexanenitrile

Under an atmosphere of nitrogen, the Grignard reagent is prepared from 1-chloro-4-benzyloxy-butane (39.7 g, 0.2 mol), magnesium turnings (10 g, 0.4 mol), and dry ether (400 ml). The Grignard solution is separated from the excess of magnesium, transferred to a 2 l flask, and cooled to −40°C. A solution of fluoroacetonitrile (10.6 g, 0.18 mol) in ether (100 ml) is added slowly, the temperature being maintained between −40 and −30°C. Stirring is continued for 30 minutes more at this temperature. The reaction mixture is then poured into a vigorously stirred solution of sodium cyanide (39 g) and ammonium chloride (59 g) in water (200 ml) containing some ice (200 g). The mixture is vigorously stirred and allowed to warm up to room temperature during one hour. The organic phase is separated and extracted with 1 N HCl (2 x 250 ml). Basification with concentrated ammonia, extraction with ether, drying, and evaporation gives the title compound as a brown oil (28.3 g).

NMR ($CDCl_3$) $\delta$:
1.63 (6H +2 $NH_2$, m + broad s), 3.48 (2H, m), 4.27 (2H, ABX, $J_{AB}$=9Hz, $J_{AX}$=$J_{BX}$=$J_{H-F}$=47Hz), 4.48 (2H, s), 7.33 (5H, s).

The product can be used for the next step without further purification.

D) 2-Fluoromethyl-2-phthalimido-6-benzyloxy-hexanenitrile

A solution of 2-fluoromethyl-2-amino-6-benzyloxy-hexanenitrile (28.3 g, 113 mol) and triethylamine (34.4 g) in dry dichloromethane (200 ml) is cooled in an ice bath, and a solution of phthaloyl dichloride (20.7 g) in dichloromethane (100 ml) is added slowly. After stirring at room temperature overnight, the solution is washed with 2 N HCl and with water. The solution is dried, treated with charcoal, and evaporated to give a brown oil (37 g). Flash chromatography on silica gel (1 kg, ether/petroleum ether 30:70) gives the pure title compound (16 g).

NMR ($CDCl_3$) $\delta$:
1.70 (6H, m), 3.47 (2H, broadened t), 4.47 (2H, s), 5.07 (2H, ABX, $J_{AB}$=9Hz, $J_{AX}$=$J_{BX}$=$J_{H-F}$=47Hz), 7.32 (5H, s), 7.87 (4H, s).

E) 2-Fluoromethyl-2-phthalimido-6-hydroxy-hexanenitrile

A solution of 2-fluoromethyl-2-phthalimido-6-benzyloxy-hexanenitrile (15.5 g, 40.8 mmol) and trimethylsilyliodide (13 ml, 2.2 equivalents) in dry dichloromethane (100 ml) is stirred at room temperature under nitrogen overnight. After removing the solvent under vacuum, the residue is dissolved in dry chloroform, triethylamine (17 ml, 3 equivalents) is added, and the mixture is refluxed for 30 minutes. Upon cooling, the solution is stirred with 2 N HCl (250 ml) for 15 minutes, the phases are separated, and the organic layer is washed with sodium bicarbonate and with water. After evaporation, the residue is dissolved in THF and water (20 ml). Drops of 6 N HCl are added, and the resulting mixture is stirred for several minutes. The solvent is removed by evaporation, and the residue obtained is dissolved in chloroform, washed with water, and dried. Removal of solvent gives the crude title compound as an oil. (12 g). Chromatography on silica (300 g, eluent: ethylacetate) gives the pure material (9.0 g).

NMR ($CDCl_3$) $\delta$:
1.25—2.0 +2.0—3.0 (7H, 2m), 3.57 (2H, broadened t), 5.03 (2H, ABX, $J_{AB}$=9Hz, $J_{AX}$=$J_{BX}$=$J_{H-F}$=46Hz), 7.80 (4H, s).

F) 2-Fluoromethyl-2-phthalimido-6-methanesulfonyloxy-hexanenitrile

A solution of 2-fluoromethyl-2-phthalimido-6-hydroxy-hexanenitrile (9.0 g, 31 mmol) and pyridine (60 ml) in dry dichloromethane (150 ml) is cooled in an ice bath, and methanesulfonyl chloride (3.6 g, 31 mmol), diluted with a small amount of dichloromethane, is added slowly with efficient stirring. Stirring is continued at room temperature overnight. The reaction mixture is washed with 2 N HCl and dried. Evaporation of solvent gives the title compound as a yellow oil (10.84 g).

NMR ($CDCl_3$) $\delta$:
1.83 (6H, m), 3.00 (3H, s), 4.23 (2H, broadened t), 5.00 (2H, ABX, $J_{AB}$=9Hz, $J_{AX}$=$J_{BX}$=$J_{H-F}$=46Hz), 7.83 (4H, s).

G) 2-Fluoromethyl-2-phthalimido-6-iodo-hexanenitrile

2-Fluoromethyl-2-phthalimido-6-methanesulfonyl-oxy-hexanenitrile (10.84 g, 29.5 mmol) and sodium iodide (8.8 g, 2 equivalents) are refluxed in acetone overnight. The reaction mixture is filtered and solvent is removed by evaporation. The residue obtained is dissolved in ether and washed with water, sodium bisulfite, and water again. Upon concentration, the title compound crystallizes (9.0 g).

NMR ($CDCl_3$) $\delta$:
1.4—2.6 (6H, m), 3.18 (2H, broadened t), 5.02 (2H, ABX, $J_{AB}$=9Hz, $J_{AX}$=$J_{BX}$=$J_{H-F}$=46Hz), 7.86 (4H, s).

H) 2-Fluoromethyl-2,6-diphthalimido-hexanenitrile

A mixture of 2-fluoromethyl-2-phthalimido-6-iodo-hexanenitrile (9.0 g, 22.5 mmol), dry DMF (30 ml), and potassium phthalimide (4.2 g, 1 equivalent) is stirred and heated at 80°C for 4 hours. The DMF is removed under reduced pressure (0.1 mm Hg), and the residue is taken up in chloroform. The chloroform mixture is filtered, and the filtrate is washed with 2 N NaOH, 2 N HCl, and water. Evaporation of solvent gives a residue which, when dissolved in the minimum amount of acetone, crystallizes upon addition of ether. After standing at 5°C overnight, the colourless crystals are collected (7.0 g), mp (Kofler) 136°C.

NMR ($CDCl_3$) $\delta$:
1.2—3.0 (6H, m), 3.70 (2H, broadened t), 5.07 (2H, ABX, $J_{AB}$=9Hz, $J_{AX}$=$J_{BX}$=$J_{H-F}$=46Hz), 7.77 + 7.83 (8H, 2s).

I) 2-Fluoromethyl-2,6-diamino-hexanoic acid

2-Fluoromethyl-2,6-diphthalimido-hexanenitrile (5.0 g, 11.9 mmol) is refluxed with concentrated HCl for 14 hours. After addition of more concentrated HCl, heating is continued for 18 hours more. After the reaction mixture is cooled to room temperature, phthalic acid is removed by filtration, and the filtrate is evaporated to dryness. The residue is dissolved in water, treated with charcoal, and extracted three times with ether. Solvent is removed by evaporation. The residue is dried carefully (oil pump) and dissolved in dry ethanol. Ammonium chloride is removed by filtration, and the filtrate is treated with an excess of propylene oxide. After 2 hours at room temperature, the precipitate is removed by filtration and washed with ethanol and ether to give 2.6 g of crude material. This material is dissolved in the minimum amount of water. The water solution is filtered and upon addition of ethanol to the filtrate, the title compound crystallizes (1.0 g). A second crop (0.9 g) is obtained from the mother liquor. This second crop is recrystallized once more in the same manner. The total yield of pure title compound as the monohydrochloride: 1.50 g, mp 212°C.

Analysis for $C_7H_{15}FN_2O_2$, HCl:

| | |
|---|---|
| Calculated: | C, 39.17; H, 7.51; N, 13.05; % |
| Found: | C, 39.33; H, 7.14; N, 13.04; % |

NMR ($D_2O$/DCl) $\delta$:
1.2—2.3 (6H, m), 3.09 (2H, broad t), 4.91 (2H, ABX, $J_{AB}$=10Hz, $J_{AX}$=$J_{BX}$=$J_{H-F}$=46Hz).

## Example 10

*2-Difluoromethyl-2-amino-5-benzyloxy-pentanenitrile*

Under an atmosphere of nitrogen, 3-benzyloxypropyl-magnesium chloride is prepared from 1-chloro-3-benzyloxypropane (2.42 g, 13 mmol) in dry ether (13 ml) and magnesium turnings (660 mg, 26 mmol). The Grignard solution is separated from the excess of magnesium, cooled to −40°C, and a 10% solution of difluoroacetonitrile in dry THF (10 ml, 13 mmol) is added dropwise, maintaining the temperature at −40°C. After stirring at this temperature, for 30 minutes, the mixture is poured into a cold (0°C) solution of sodium cyanide (1.27 g) and ammonium chloride (2.10 g) in water (40 ml). The mixture is stirred vigorously for 1 hour; during that time the reaction is allowed to warm up to room temperature. The organic phase is separated and extracted with 2 N HCl. Basification with concentrated ammonia (pH ~ 9—10), extraction with ether, drying, and evaporation gives the title compound as an oil (1.5 g).

NMR ($CDCl_3$) $\delta$:
1.90 (4H, +$NH_2$, m), 3.50 (2H, m), 4.48 (2H, s), 5.53 (1H, t, $J_{H-F}$=56Hz), 7.30 (5H, s).

Example 11

*Preparation of 2-fluoromethyl-2,5-diamino-4-oxo-valeric acid*

A) 2-Fluoromethyl-2-amino-4-methyl-4-pentene-nitrile

In a 10 L reactor, filled with nitrogen, about 100 mL of a solution of methallyl chloride (453 g, 490 mL, 5.0 moles) in dry tetrahydrofuran (THF) (4 L) is added to a stirred suspension of magnesium turnings (486 g, 20 moles) in THF (1 L), previously activated by 2 mL of methyl iodide. The mixture is heated until Grignard formation starts, then the reactor is cooled with ice and methallyl chloride solution is added at such a rate that the internal temperature does not exceed 50°C. After stirring overnight at room temperature, the Grignard is separated from the excess of magnesium, transferred to a 20 L reactor, and cooled to —40°C. A solution of fluoroacetonitrile (276 g, 253 mL, 4.68 moles) in THF (1 L) is added slowly (within about 15 min), maintaining the internal temperature between —40 and —35°C. Stirring is continued for 30 minutes at —40°C, then the mixture is cooled to —60°C and hydrolyzed by slow addition of a water/THF mixture (300 mL, 1:1). After that, a solution of ammonium chloride (795 g) and sodium cyanide (490 g) in water (7.5 L), previously cooled with ice, is poured in rapidly, the dry ice bath is removed, and the mixture is stirred for 1 hour at an internal temperature between 0°C and room temperature. After saturation with sodium chloride (about 2 kg), the organic layer is separated, and the aqueous phase is extracted twice with ether (2 x 3 L). Drying ($Na_2SO_4$) and evaporation gives a dark oil (687 g) which is dissolved in ether (5 L) and extracted carefully with 10% hydrochloric acid (4 x 650 mL). The combined aqueous phases are cooled with ice and made basic with conc. ammonia. The oil which separates is dissolved in diethyl ether (2.5 L), and the aqueous layer is extracted with diethyl ether (2 x 2 L). Drying ($Na_2SO_4$) and evaporation gives the crude title compound as a dark oil (488 g) which can be used for the next step without further purification.

NMR ($CDCl_3$):

1.93 (3H, s), 2.37 (2H, AB, $J_{AB}$=13Hz), 4.33 (2H, ABX, $J_{AB}$=8Hz, $J_{AX}$=$J_{BX}$=$J_{H-F}$), 5.0 (2H, m).

B) 2-Fluoromethyl-2-phthalimido-4-methyl-4-pentenenitrile

In a 10 L reactor, equipped with a drying tube ($CaCl_2$), a solution of 2-fluoromethyl-2-amino-4-methyl-4-pentenenitrile obtained as in step A above (488 g, 3.44 moles) and triethylamine (685 g, 6.78 moles) in dry dichloromethane is cooled in an ice bath. A solution of phthaloyldichloride (625 g, 3.1 moles) in dichloromethane (1 L) is added slowly with stirring. After removal of the ice bath, the mixture is stirred at room temperature overnight. The reaction mixture is washed with 2 N hydrochloric acid (2 x 2 L), and then with water (2 x 2 L). The organic phase is dried ($Na_2SO_4$), and evaporated to give a residue. The residue is dissolved in dry dichloromethane (4 L), triethylamine (200 mL) is added, and the resulting mixture is refluxed for 4 hours (internal temperature 42°C). Work-up as previously described gives an oil which solidifies on standing (773 g).

The solidified oil (60 g portions) is treated in a mortar with ethanol (45 mL). The mixture is filtered, washed with ethanol (15 mL) and twice with petroleum ether to give 427 g of a yellow solid which is dissolved in benzene (1.3 L), and petroleum ether (2.2 L) is added. After several hours, more petroleum ether is added (1 L), and the mixture is kept at room temperature overnight. Filtration gives pure title material (349 g) (single spot on this layer chromatography). A second crop is obtained by concentrating the mother liquor. The mother liquor of this second crystallisation is combined with the filtrate of the ethanol washings, evaporated, and chromatographed on silica (2 kg, AcOEt/PE 20:80) to give an additional amount of pure material. Total yield: 471 g.

NMR ($CDCl_3$)

1.88 (3H, s), 2.98 (2H, AB, $J_{AB'}$=13Hz), 4.85 (2H, m), 5.17 (2H, ABX, $J_{AB}$=9Hz, $J_{AX}$=$J_{BX}$=$J_{H-F}$=46Hz), 7.80 (4H, s).

C) 2-Fluoromethyl-2-phthalimido-4-bromomethyl-4-pentenenitrile

2-Fluoromethyl-2-phthalimido-4-methyl-4-pentenenitrile obtained as in Step B above (12.38 g, 45.4 mmoles), N-Bromosuccinimide (8.11 g, 45.6 mmoles), dry $CCl_4$ (100 mL), and a few mgs of benzoylperoxide are heated under reflux by irradiation with a lamp (375 W) for 4 1/2 hours. Every hour, a few more mgs of benzoylperoxide are added. The reaction is monitored by NMR. After 4 1/2 hours, less than 10% of starting material is left. After cooling to room temperature, succinimide is filtered off. The filtrate is washed with water (3 x 100 mL), dried ($Na_2SO_4$) and evaporated. The crude title compound is obtained as a solid (14.94 g) which can be used for the next step without further purification.

NMR ($CDCl_3$)

3.20 (2H, AB, $J_{AB}$=13Hz), 4.10 (2H, AB, $J_{AB}$=11Hz), 5.10 (1H, s), 5.13 (2H, ABX, $J_{AB}$=9Hz, $J_{AX}$=$J_{BX}$=$J_{H-F}$=46Hz), 5.37 (1H, s), 7.73 (4H, s).

D) 2-Fluoromethyl-2-phthalimido-4-phthalimidomethyl-4-pentene-nitrile

2-Fluoromethyl-2-phthalimido-4-bromomethyl-4-pentene-nitrile obtained as in Step C above (14.94 g, 42.6 mmoles), potassium phthalimide (7.90 g, 42.7 mmoles) and dry dimethylformamide (DMF) (100 mL, refluxed over and distilled from calcium hydride) are heated (bath temperature 70—80°C) for 3 hours. The DMF is removed under vacuum (oil pump), the residue is dissolved in chloroform, salts are removed by filtration, and the solution is washed with 1 N sodium hydroxide and several times with water. The washed filtrate is dried ($Na_2SO_4$) and evaporated to give the crude title compound as a viscous oil. The oil is dissolved in chloroform (minimum amount). Equal volumes of diethyl ether and petroleum ether are added. Upon standing overnight, crystals (5.0 g) from the oil are collected. The filtrate is evaporated, and the residue is chromatographed on silica (35 g/kg; AcOEt/PE 40:60). Total yield of pure title compound: 8.83 g.

NMR ($CDCl_3$)

3.17 (2H, AB, $J_{AB}$=14Hz), 4.33 (2H, s), 5.17 (2H, s with fine splitting), 5.23 (2H, ABX, $J_{AB}$=9Hz, $J_{AX}$=$J_{BX}$=$J_{H-F}$=46Hz), 7.82 (8H, s with fine splitting).

E) 2-Fluoromethyl-2,5-diphthalimido-4-oxopentane-nitrile

A solution of 2-Fluoromethyl-2-phthalimido-4-phthalimido-methyl-4-pentene nitrile obtained as in Step D above (2.65 g, 6.32 mmoles) in a 1:1 mixture (50 mL) of methylene chloride and methanol is cooled to −78°C and treated with ozone (flow rate about 0.3 L/min) for 12 3/4 minutes (i.e., 2 min/mole). An excess of dimethylsulfide (2 mL) is added, and the mixture is allowed to warm up to room temperature. After standing for 2 hours, the insoluble title ketone (1.73 g) is collected and washed with a small amount of chloroform and ether. The ketone can be used for the next step without further purification.

An analytical sample was obtained by recrystallization from hot tetrahydrofuran (100 mL/3.5 g)/$CHCl_3$ (100 mL).

Analysis for: $C_{22}H_{14}FN_3O_5$

| | |
|---|---|
| Calculated: | C, 63.01; H, 3.37; N, 10.02 |
| Found: | C, 62.91; H, 3.61; N, 10.03 |

F) 2-Fluoromethyl-2,5-diamino-4-oxo-valeric acid, monohydrochloride

2-Fluoromethyl-2,5-diphthalimido-4-oxo-pentanenitrile obtained as in Step E above (5.78 g, 13.8 mmoles) is heated with conc. hydrochloric acid (50 mL) at 100°C (bath temperature) for 32 hours. After cooling to room temperature, phthalic acid is removed by filtration, and the filtrate is evaporated. The residue is dissolved in 1 N hydrochloric acid (50 mL) and extracted with ether (3 × 50 mL). After evaporation of the ether, the residue is dried carefully overnight (oil pump) and then dissolved in a 1:1 mixture of methanol and ethanol (80 mL). Ammonium chloride is removed by filtration and washed with the same methanol/ethanol mixture (10 mL). The washings are added to the original methanol/ethanol solution. Propylene oxide (3 mL), is added and the mixture is kept at room temperature for several hours, then in the refrigerator overnight. The crude monohydrochloride is collected, washed with a small amount of ethanol and ether, and dried (2.14 g). Treatment of the solid with charcoal in water at room temperature for 3 1/2 hours and evaporation of water gives a colourless material (2.11 g) which is recrystallized from water (15 mL) and ethanol. Drying at room temperature under vacuum (oil pump) in the presence of $P_2O_5$ gives the title compound as monohydrochloride semihydrate 1.60 g, mp 154°C.

NMR ($D_2O$/DCl):

3.53 (2H, narrow AB, $J_{AB}$=18Hz), 4.23 (2H, s), 4.87 (2H, d, $J_{H-F}$=46Hz).

Analysis for $C_6H_{11}FN_2O_3$, HCl, 1/2 $H_2O$

| | |
|---|---|
| Calculated: | C, 32.22; H, 5.86; N, 12.53 |
| Found: | C, 32.25; H, 5.83; N, 12.48 |

Example 12

*2-Fluoromethyl-2,5-diamino-4-oxo-valeric acid (α-Fluoromethyl-γ-oxo-ornithine)*

A) 2-Fluoromethyl-2-amino-4-pentenenitrile

The Grignard reagent is prepared under an atmosphere of nitrogen, from magnesium turnings (19.1 g, 0.79 mol) and allyl chloride (60 g, 0.79 mol) in dry THF (800 ml). The reaction mixture is cooled to −30°C, and fluoroacetonitrile (30.2 g, 0.51 mol) in THF (100 ml) is added slowly. The mixture is then stirred for 30 more minutes at −30°C. The reaction mixture is then poured into a solution of sodium cyanide (50 g, 1.2 mol) and ammonium chloride (81 g, 1.53 mol) in water (1.1 l),

and the mixture is stirred for 1 hour. After saturating the mixture with NaCl, the THF layer is separated and the aqueous phase is extracted with ether. Washing the ether layer with water and evaporation of solvent gives an oil (40 g), which is dissolved in ether and extracted with 2 N HCl. Basification with concentrated ammonia, re-extraction with ether, drying, and removal of the solvent gives the title compound as a brown oil (31.98 g).

NMR ($CDCl_3$) $\delta$:
1.5—2.8 (2H + $NH_2$, m + broad s), 4.37 (2H, ABX, $J_{AB}$=10Hz, $J_{AX}=J_{BX}=J_{H-F}$=47Hz), 5.00—6.28 (3H, m).

This material is used for the next step without further purification.

B) 2-Fluoromethyl-2-amino-4-pentenoic acid

2-Fluoromethyl-2-amino-4-pentenenitrile (11.4 g, 89 mmol) is refluxed with concentrated HCl for 10 hours. After evaporation of the reaction mixture to dryness, the residue is dried carefully (oil pump). It is then dissolved in isopropanol (200 ml), ammonium chloride is removed by filtration, and the crude title compound is precipitated by addition of propylene oxide (15.5 g). Yield 5.0 g.

NMR ($D_2O$/DCl) $\delta$:
2.83 (2H, d with fine splitting), 5.00 (2H, ABX, $J_{AB}$=10Hz, $J_{AX}=J_{BX}=J_{H-F}$=47Hz), 5.3—6.0 (3H, m).

C) Methyl 2-fluoromethyl-2-amino-4-pentenoate

A mixture of 2-fluoromethyl-2-amino-4-pentenoic acid (3.83 g, 26 mmol), trimethylorthoformate (4.13 g, 39 mmol), and dry methanol (110 ml) is saturated with dry HCl gas with stirring and ice cooling. After the mixture is refluxed overnight, the solvent is evaporated to give the title compound as the hydrochloride (5.0 g).

NMR ($D_2O$/DCl) $\delta$:
2.83 (2H, d with fine splitting), 3.93 (3H, s), 4.97 (2H, ABX, $J_{AB}$=11Hz, $J_{AX}=J_{BX}=J_{H-F}$=45Hz), 5.45—6.2 (3H, m).

The free amino acid ester is obtained by dissolving the hydrochloride in a small amount of water, adjusting the pH to approx. 9 with sodium carbonate, saturating the solution with NaCl, and extraction with ether. From 4.62 g of the hydrochloride, 2.10 g of the free amino acid ester is obtained:

NMR ($CDCl_3$) $\delta$:
2.80 (2H, broadened d), 3.87 (3H, s), 4.87 (2H, d, $J_{H-F}$=46Hz), 5.1—6.2 (3H, m).

D) Methyl 2-fluoromethyl-2-phthalimido-4-pentenoate

To a solution of methyl 2-fluoromethyl-2-amino-4-pentenoate (2.10 g, 13 mmol) and triethylamine (3 g) in dry dichloromethane, phthaloyl dichloride (2.37 g, 11.7 mmol) is added with ice cooling. After stirring overnight, at room temperature, the solution is washed with water and 1 N HCl. Drying and removal of solvent by evaporation gives a residue, consisting mainly of the iso-phthalimide. The residue is dissolved in dry dichloromethane, two equivalents of triethylamine are added, and the mixture is refluxed overnight. Work-up as described above gives the title compound as an oil (3.0 g).

NMR ($CDCl_3$) $\delta$:
3.17 (2H, broadened t, J=7Hz), 3.77 (3H, s), 5.10 (2H, ABX, $J_{AB}$=9Hz, $J_{AX}=J_{BX}=J_{H-F}$=47Hz), 4.9—6.2 (3H, m), 7.77 (4H, s).

E) Methyl 2-fluoromethyl-2-phthalimido-3-carboxy-propionate

A solution of methyl 2-fluoromethyl-2-phthalimido-4-pentenoate (2.8 g, 9.6 mmol) and potassium permanganate (4.56 g, 29 mmol) in acetic acid (35 ml) and water (150 ml) is stirred at room temperature overnight. Sodium bisulfite is added to dissolve manganese dioxide. Saturation with NaCl, ether extraction, and evaporation of the solvent gives the title compound as a colourless oil which crystallizes on standing (2.10 g).

NMR ($CDCl_3$) $\delta$:
3.43 (2H, m), 3.80 (3H, s), 5.27 (2H, ABX, $J_{AB}$=9Hz, $J_{AX}=J_{BX}=J_{H-F}$=47Hz), 7.77 (4H, s).

F. Methyl 2-fluoromethyl-2-phthalimido-3-chlorocarbonyl-propionate

A solution of methyl 2-fluoromethyl-2-phthalimido-3-carboxy-propionate (2.37 g, 13.7 mmol) and pyridine (1.10 g, 13.7 mmol) in dry ether (50 ml) is treated with thionyl chloride (10 ml) at room

temperature overnight. After addition of more ether (100 ml), pyridine hydrochloride is removed by filtration, and the solvent is evaporated to give the title compound as an orange oil (2.80 g).

NMR ($CDCl_3$) $\delta$:
3.80 (3H, s), 4.03 (2H, s), 5.23 (ABX, $J_{AB}$=9Hz, $J_{AX}$=$J_{BX}$=$J_{H-F}$=47Hz), 7.77 (4H, s).

G) Methyl 2-fluoromethyl-2-phthalimido-4-oxo-5-diazo-pentanoate

A solution of methyl 2-fluoromethyl-2-phthalimido-3-chlorocarbonyl-propionate (2.07 g, 6.33 mmol) in dry ether (24 ml) is slowly added with stirring to a cooled (ice) solution of diazomethane in ether (0.392 mol, 50 ml). After 30 minutes, the solvent is evaporated to give a yellow oil (2.1 g). This is purified by flash chromatography on silica gel (200 g, ethyl acetate/petroleum ether 4:6) to give the pure title compound as a yellow oil (1.7 g);

NMR ($CDCl_3$) $\delta$:
3.33 (2H, AB, $J_{AB}$=16Hz, additional fine splitting, ~ 1.5Hz), 3.80 (3H, s), 5.27 (2H, ABX, $J_{AB}$=9Hz, $J_{AX}$=$J_{BX}$=$J_{H-F}$=47Hz), 5.40 (1H, s), 7.77 (4H, s).

H) Methyl 2-fluoromethyl-2-phthalimido-4-oxo-5-bromo-pentanoate

A solution of methyl 2-fluoromethyl-2-phthalimido-4-oxo-5-diazo-pentanoate (1.53 g, 4.62 mmol) in ether (22.5 ml) is added dropwise and with vigorous stirring to cold (ice cooling) aqueous HBr (47%, 0.95 ml). Stirring is continued for 1 hour at room temperature. The organic phase is separated, washed with water, dried, and evaporated to give the title compound as a yellow oil (1.52 g).

NMR ($CDCl_3$) $\delta$:
3.66 (2H, s with fine splitting), 3.80 (3H, s), 4.07 (2H, s), 5.20 (2H, ABX, $J_{AB}$=9Hz, $J_{AX}$=$J_{BX}$=$J_{H-F}$=47Hz), 7.75 (4H, s).

I) 2-Fluoromethyl-2,5-diamino-4-oxo-valeric acid

A mixture of methyl 2-fluoromethyl-2-phthalimido-4-oxo-5-bromo-pentanoate (1.42 g, 3.69 mmol), potassium phthalimide (820 g, 4.4 mmol) in dry DMF (5 ml) is heated at 70°C for 3 hours. The solvent is removed under vacuum (oil pump), and the residue is refluxed with concentrated HCl for 32 hours. Work-up and propylene oxide precipitation as described previously gives the crude title product as the monohydrochloride (540 mg).

Example 13

*2-Fluoromethyl-2-amino-3-(4'-imidazolyl)propionic acid (α-Fluoromethylhistidine)*

A) 2-Fluoromethyl-2-amino-3-[4'-(2'-mercapto)-imidazolyl]-propionic acid

A solution of 2-fluoromethyl-2,5-diamino-4-oxo-hexanoic acid monohydrochloride semihydrate (60.0 g, 0.268 mol) and potassium thiocyante (140 g, 1.44 mol) in water (67 ml) is stirred and heated at 100°C for 3 hours. The mixture is then allowed to cool to room temperature and kept at 5°C for several hours. The title compound is collected and washed with water, ethanol, and ether. Yield 43.03 g. This material, mp 271°C (dec.) is used for the next step without further purification.

NMR ($D_2O$/DCl) $\delta$:
3.53 (2H, s), 5.06 (2H, ABX, $J_{AB}$=11Hz, $J_{AX}$=$J_{BX}$=$J_{H-F}$=45Hz), 7.23 (1H, s).

B) 2-Fluoromethyl-2-amino-3-(4'-imidazolyl)-propionic acid

A solution of 2-fluoromethyl-2-amino-3-[4'-(2'-mercapto)]-propionic acid (43.03 g, 0.196 mol) and ferric chloride (159.5 g, 0.984 mol) in 2 N HCl (100 ml) and water (2 l) is heated for 2 hours (bath temperature: 110°C). The reaction mixture is cooled to room temperature, sodium acetate trihydrate (535 g) and water (2 l) are added, and the solution is saturated with hydrogen sulfide. After filtration and carefully washing with water, the filtrate is acidified strongly with concentrated HCl and evaporated to dryness. The residue is dissolved in concentrated HCl (2 l). Sodium chloride is removed by filtration and washed with concentrated HCl (3 × 500 ml). Solvent is removed and the residue is dissolved in water (1.4 l). Concentrated ammonia is added until a pH 9—10 (about 30 ml) is achieved. After addition of more ammonia (10 ml), the solution is saturated with hydrogen sulfide again. After filtration, the solution is acidified (HCl) to precipitate some sulfur, which is removed by filtration through a membrane filter (Millipore)®. At this stage, the solution is tested qualitatively for the absence of ferrous and ferric ions (oxidation and thiocyanate testing).

Solvent is removed by filtration, and the residue is dissolved in water (1.4 l). A 20% aqueous solution of barium chloride is added in portions until the supernatant gives a precipitate with sodium sulfate solution (total amount of $BaCl_2$-solution = 190 ml). After removing $BaSO_4$ by filtration, concentrated HCl is added, and the solution is evaporated to dryness. The residue is dried carefully overnight under vacuum (oil pump). It is then dissolved in dry ethanol (1.2 l). Ammonium chloride (and

excess barium chloride) are removed from the solution by filtration. Crude α-fluoromethylhistidine, monohydrochloride, is precipitated by addition of propylene oxide (40 ml). Systematic work-up of the mother liquors (evaporation with HCl, reprecipitation with propylene oxide) gives a total of 36.9 g of crude material which, according to NMR, retains some ethanol. This material is dissolved in water, treated with charcoal (10 weight-%) at room temperature and crystallized by addition of isopropanol to give 16.33 g of product. A second recrystallization (5% charcoal) gives a first crop (10.51 g) of pure title compound. More pure material is obtained by systematic work-up: mother liquors of the first crystallization are converted to the di-hydrochloride and re-precipitated with propylene oxide; mother liquors of the second crystallization are treated with charcoal and recrystallized from water (1.7 times the weight of compound) and isopropanol. Total yield of pure title compound as the monohydrochloride is 21 g, mp 218°C (dec.).

Analysis for $C_7H_{10}FN_3O_2$, HCl:

| | |
|---|---|
| Calculated: | C, 37.59; H, 4.69; N, 18.79; % |
| Found: | C, 37.42; H, 4.74; N, 18.87; % |

NMR ($D_2O$/DCl) δ:
3.40 (2H, s), 4.80 (2H, ABX, $J_{AB}$=11Hz, $J_{AX}$=$J_{BX}$=$J_{H-F}$=46Hz), 7.37 (1H, s), 8.56 (1H, s).

Example 14

*2-Fluoromethyl-2-amino-5-hexenenitrile*

Under an atmosphere of nitrogen, butenylmagnesium-bromide is prepared from magnesium turnings (3.27 g, 140 mmol) and 4-bromo-1-butene (19 g, 140 mmol) in dry ether (140 ml). After cooling to −30°C, fluoroacetonitrile (7 g, 0.12 mol) in ether (40 ml) is added slowly, and stirring is continued for 30 minutes more at −30°C, during which time the mixture becomes a viscous mass. A solution of water (1 equivalent) in methanol is added dropwise, maintaining the temperature at −30°C. A solution of sodium cyanide (3 equivalents) and ammonium chloride (4 equivalents) in water (200 ml) is then added, and the mixture is stirred vigorously for 1 hour at room temperature. Separation of the phases and extraction (ether) followed by evaporation gives a brown oil (13 g). The oil is dissolved in ether and extracted with 6 N HCl. Basification with concentrated ammonia (pH approx. 9), extraction with ether, and evaporation gives the title compound (9.1 g), which is used for the next step without further purification.

NMR ($CDCl_3$) δ:
1.5—3.0 (4H + $NH_2$, m), 4.37 (2H, ABX, $J_{AB}$=9Hz, $J_{AX}$=$J_{BX}$=$J_{H-F}$=46Hz), 4.9—6.3 (3H, m).

**Claims**

1. A process for preparing a 2-fluorinated methyl aminoacetonitrile which comprises treating a corresponding fluorinated methyl ketimine magnesium halide with hydrogen cyanide or with an alkali metal cyanide or ammonium cyanide and a proton source.

2. A process as claimed in Claim 1 wherein the reaction is carried out in an aqueous medium.

3. A process as claimed in Claim 2 wherein the proton source is a water-soluble ammonium salt of a strong acid.

4. A process as claimed in Claim 3 wherein the said ammonium salt is ammonium chloride.

5. A process as claimed in any one of the preceding Claims wherein the ketimine salt is prepared in tetrahydrofuran by reaction of the corresponding Grignard reagent with a fluorinated acetonitrile and the reaction mixture thus formed is added directly to an acidic water solution of an alkali metal cyanide or of ammonium cyanide.

6. A process as claimed in any one of the preceding Claims wherein the fluorinated methyl group is monofluoromethyl.

7. A process as claimed in any one of Claims 1 to 5 wherein the fluorinated methyl group is difluoromethyl.

8. A process as claimed in any one of the preceding Claims for preparing a 2-fluorinated methyl aminoacetonitrile of the following general formula I:

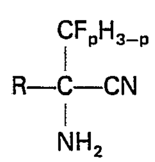

Formula I

wherein:—

R represents an organic group which is inert under the process conditions; and
*p* represents 1 or 2;

which comprises treating a ketimine magnesium halide of the following general Formula II:—

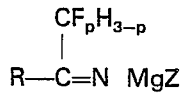

Formula II

wherein:

R and *p* are as defined above; and

Z represents bromine, chlorine or iodine;

with hydrogen cyanide or with an alkali metal cyanide or ammonium cyanide and a proton source.

9. A process as claimed in Claim 8 wherein the organic group R is an alkyl or alkenyl group optionally substituted by alkoxy, phenylalkoxy, alkenyloxy, phenyl, alkoxyphenyl or benzyloxyphenyl.

10. A process as claimed in any one of the preceding Claims for preparing a compound of the formula:

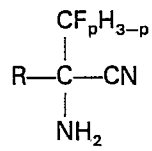

I

wherein:

*p* is 1 or 2

and R is:

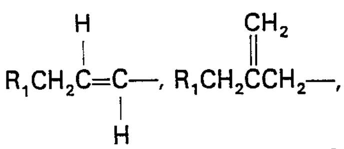

, $R_2OCH_2CH_2CH_2$—, $R_2OCH_2CH_2CH_2CH_2$—, $R_3CH_2$—, $CH_2{=}CHCH_2$—,

$CH_2{=}CHCH_2CH_2$—, or $CH_3CH{=}CHCH_2CH_2$—

wherein:

$R_1$ is hydrogen, methoxy, benzyloxy, diphenylmethoxy, triphenylmethoxy, or allyloxy;

$R_2$ is methyl, benzyl, diphenylmethyl, triphenylmethyl, or allyl; and

$R_3$ is a substituted-phenyl group of the formula:

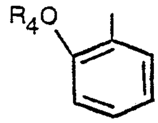 , 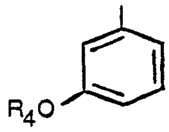 , 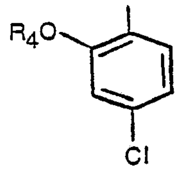 , 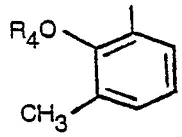 , 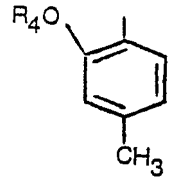 ,

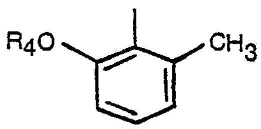 , 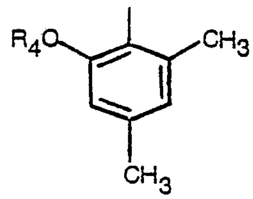 , 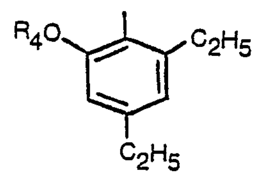 ,

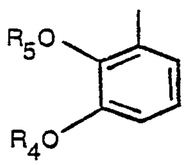 , 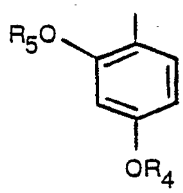 , 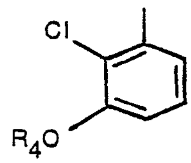 , 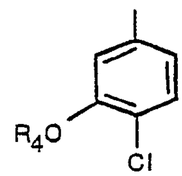 ,

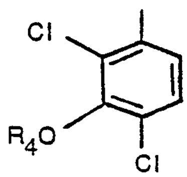 , 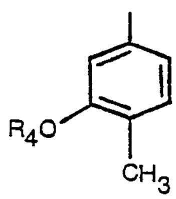 , 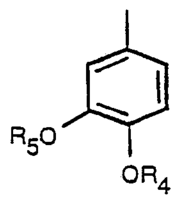 , 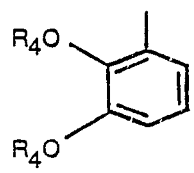 ,

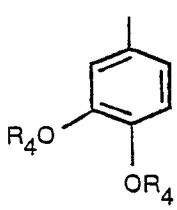 , 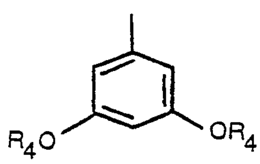 , 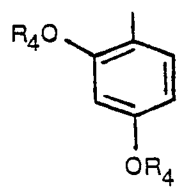 , 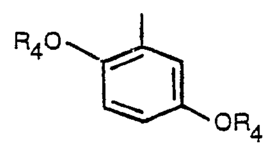 ,

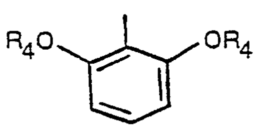 , 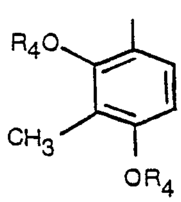 , 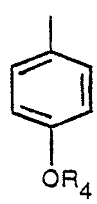 , 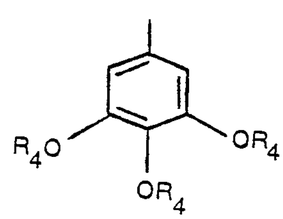 , or

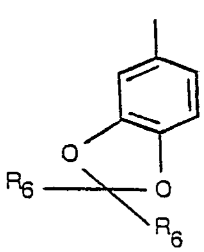 ;

wherein

$R_4$ is $C_1$—$C_8$ alkyl, $R_5$ is benzyl, and $R_6$ is hydrogen or methyl; with the proviso that when R is $R_3CH_2$— wherein $R_3$ is

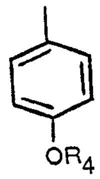

*p* cannot be 2;

which comprises treating a ketimine magnesium halide of the formula:

$$\begin{array}{c} CF_pH_{3-p} \\ | \\ R—C{=}N—MgZ; \end{array} \qquad II$$

wherein R and *p* have the meanings hereinabove defined and Z is chloride, bromide, or iodide; with hydrogen cyanide or with an alkali metal cyanide or ammonium cyanide and a proton source.

**Patentansprüche**

1. Ein Verfahren zur Herstellung eines in 2-Stellung durch eine fluorierte Methylgruppe substituierten Aminoacetonitrils, das Behandlung eines entsprechenden, mit einer fluorierten Methylgruppe substituierten Ketiminmagnesiumhalogenids mit Cyanwasserstoff oder mit einem Alkalimetallcyanid oder Ammoniumcyanid und einer Protonenquelle umfaßt.

2. Verfahren nach Anspruch 1, wobei die Umsetzung in einem wäßrigen Medium durchgeführt wird.

3. Verfahren nach Anspruch 2, wobei die Protonenquelle ein wasserlösliches Ammoniumsalz einer starken Säure ist.

4. Verfahren nach Anspruch 3, wobei das Ammoniumsalz Ammoniumchlorid ist.

5. Verfahren nach einem der vorangehenden Ansprüche, wobei das Ketiminsalz in Tetrahydrofuran durch Umsetzung des entsprechenden Grignard-Reagens mit einem fluorierten Acetonitril hergestellt wird und das so erhaltene Reaktionsgemisch direkt zu einer sauren wäßrigen Lösung eines Alkalimetallcyanids oder von Ammoniumcyanid gegeben wird.

6. Verfahren nach einem der vorangehenden Ansprüche, wobei die fluorierte Methylgruppe Monofluormethyl ist.

7. Verfahren nach einem der Ansprüche 1 bis 5, wobei die fluorierte Methylgruppe Difluormethyl ist.

8. Verfahren nach einem der vorangehenden Ansprüche zur Herstellung eines in 2-Stellung durch eine fluorierte Methylgruppe substituierten Aminoacetonitrils der folgenden allgemeinen Formel (I)

$$\begin{array}{c} CF_pH_{3-p} \\ | \\ R—C—CN \\ | \\ NH_2 \end{array} \qquad \text{Formel I}$$

in der

R einen organischen Rest bedeutet, der unter den Verfahrensbedingungen inert ist und
p 1 oder 2 darstellt,

das Behandeln eines Ketimin-magnesiumhalogenids der folgenden allgemeinen Formel II

$$\begin{array}{c} CF_pH_{3-p} \\ | \\ R—C{=}N\ MgZ \end{array} \qquad \text{Formel II}$$

in der

R und p wie vorstehend definiert sind und
Z Brom, Chlor oder Jod bedeutet,

mit Cyanwasserstoff oder mit einem Alkalimetallcyanid oder Ammoniumcyanid und einer Protonenquelle umfaßt.

9. Verfahren nach Anspruch 8, wobei der organische Rest R ein gegebenenfalls durch Alkoxy-, Phenylalkoxy-, Alkenyloxy-, Phenyl-, Alkoxyphenyl- oder Benzyloxyphenyl substituierter Alkyl- oder Alkenylrest ist.

10. Verfahren nach einem der vorangehenden Ansprüche zur Herstellung einer Verbindung der Formel

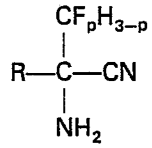

I

in der

p 1 oder 2 ist und

R einen der Reste:

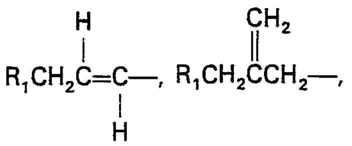 $R_2OCH_2CH_2CH_2$—, $R_2OCH_2CH_2CH_2CH_2$—, $R_3CH_2$—, $CH_2$=$CHCH_2$—,

$CH_2$=$CHCH_2CH_2$—, oder $CH_3CH$=$CHCH_2CH_2$—

bedeutet, wobei

$R_1$ Wasserstoff, Methoxy, Benzyloxy, Diphenylmethoxy, Triphenylmethoxy oder Allyloxy ist,

$R_2$ Methyl, Benzyl, Diphenylmethyl, Triphenylmethyl oder Allyl ist, und

$R_3$ eine substituierte Phenylgruppe der Formel

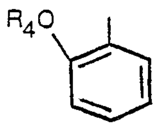 , 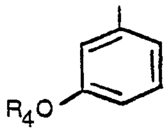 , 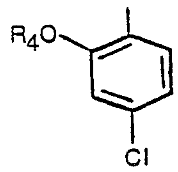 , 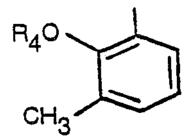 , 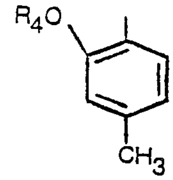 ,

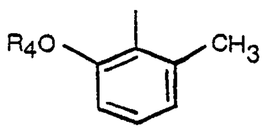 , 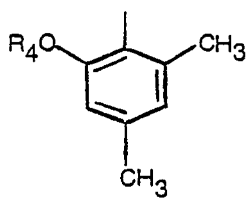 , 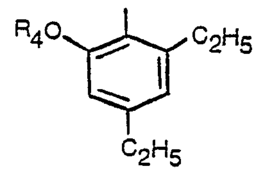 ,

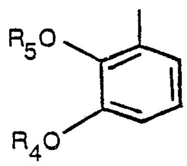 , 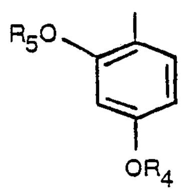 , 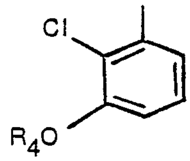 , 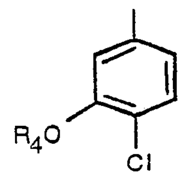 ,

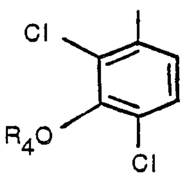 , 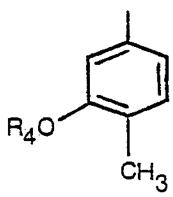 , 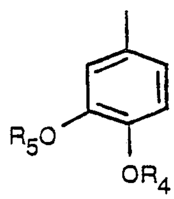 , 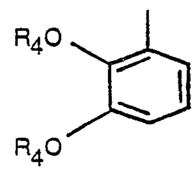 ,

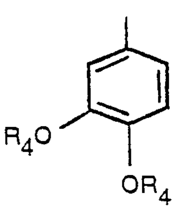 , 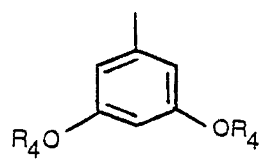 , 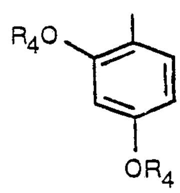 , 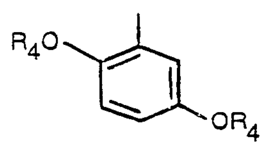 ,

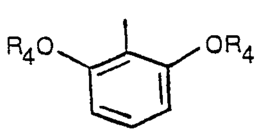 , 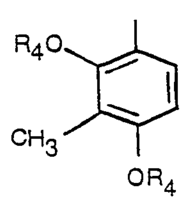 , 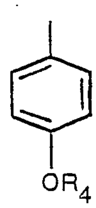 , 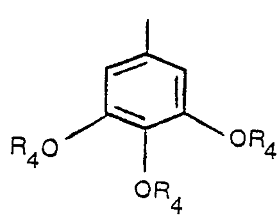 , oder

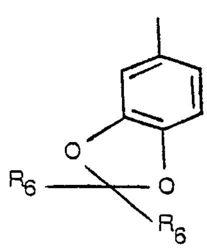 ;

darstellt,
wobei $R_4$ $C_1$—$C_8$-Alkyl ist, $R_5$ Benzyl ist und $R_6$ Wasserstoff oder Methyl ist, mit der Maßgabe, daß p nicht 2 sein kann, wenn R den Rest $R_3CH_2$— bedeutet, in dem $R_3$

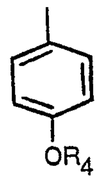

ist, das Behandlung eines Ketimin-magnesiumhalogenids der Formel

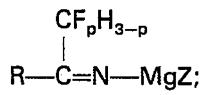

II

in der R und p die vorstehend angegebenen Bedeutungen haben und Z Chlorid, Bromid oder Jodid ist, mit Cyanwasserstoff oder mit einem Alkalimetallcyanid oder Ammoniumcyanid und einer Protonenquelle umfaßt.

**Revendications**

1. Un procédé pour préparer un 2-(méthyl fluoré)aminoacétonitrile qui consiste à traiter un halogénure de (méthyl fluoré)-cétimine-magnésium correspondant par le cyanure d'hydrogène ou par un cyanure de métal alcalin ou le cyanure d'ammonium et une source de protons.

2. Un procédé selon la revendication 1, dans lequel la réaction est effectuée en milieu aqueux.

3. Un procédé selon la revendication 2, dans lequel la source de protons est un sel d'ammonium soluble dans l'eau d'un acide fort.

4. Un procédé selon la revendication 3 dans lequel ledit sel d'ammonium est le chlorure d'ammonium.

5. Un procédé selon l'une quelconque des revendications précédents, dans lequel le sel de cétimine est préparé dans le tétrahydrofuranne par réaction du réactif de Grignard correspondant avec un acétonitrile fluoré et le mélange de réaction ainsi formé est ajouté directement à une solution aqueuse acide d'un cyanure de métal alcalin ou de cyanure d'ammonium.

6. Un procédé selon l'une quelconque des revendications précédentes, dans lequel le groupe méthyle fluoré est le groupe monofluorométhyle.

7. Un procédé selon l'une quelconque des revendications 1 à 5 dans lequel le groupe méthyle fluoré est le groupe difluorométhyle.

8. Un procédé selon l'une quelconque des revendications précédentes pour préparer un 2-(méthyl fluoré)aminoacétonitrile de formule générale I:

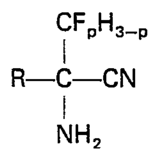

formule I

dans laquelle
R représente un groupe organique inerte dans les conditions du procédé; et
p représente 1 ou 2;
qui consiste à traiter un halogénure de cétimine-magnésium de formule générale II:

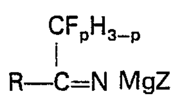

formule II

dans laquelle
R et p sont comme définis ci-dessus; et
Z représente le brome, le chlore ou l'iode;

avec le cyanure d'hydrogène ou avec un cyanure de métal alcalin ou le cyanure d'ammonium et une source de protons.

9. Un procédé selon la revendication 8, dans lequel le groupe organique R est un groupe alkyle ou alcényle, facultativement substitué par alcoxy, phénylalcoxy, alcényloxy, phényle, alcoxyphényle ou benzyloxyphényle.

10. Un procédé selon l'une quelconque des revendications précédentes, pour préparer un composé de formule:

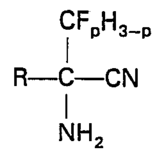 I

dans laquelle p est 1 ou 2
et R est:

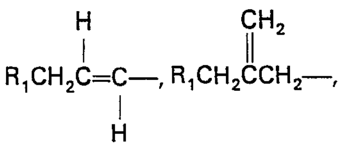 $R_2OCH_2CH_2CH_2$—, $R_2OCH_2CH_2CH_2CH_2$—, $R_3CH_2$—, $CH_2{=}CHCH_2$—,

$CH_2{=}CHCH_2CH_2$—, ou $CH_3CH{=}CHCH_2CH_2$—

où

$R_1$ est l'hydrogène, méthoxy, benzyloxy, diphénylméthoxy, triphénylméthoxy ou allyloxy;
$R_2$ est méthyle, benzyle, diphénylméthyle, triphénylméthyle ou allyle; et
$R_3$ est un groupe phényle substitué de formule:

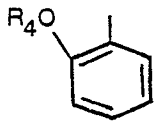 , 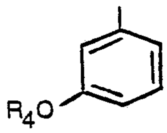 , 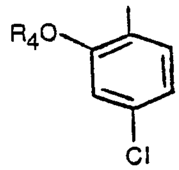 , 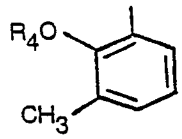 , 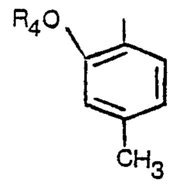

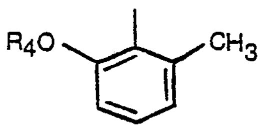 , 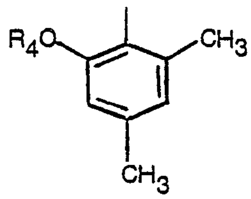 , 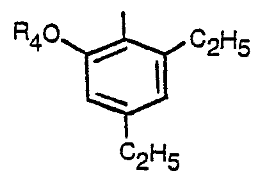 ,

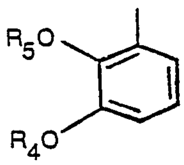 , 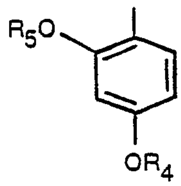 , 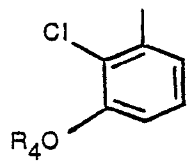 , 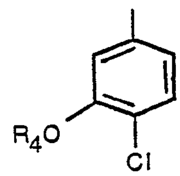 ,

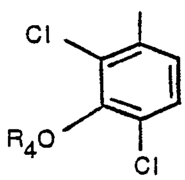 , 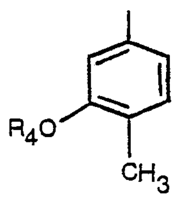 , 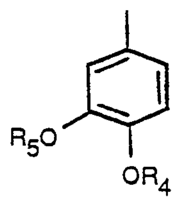 , 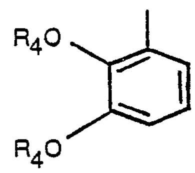

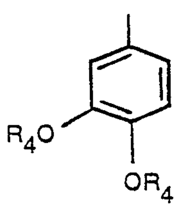 , 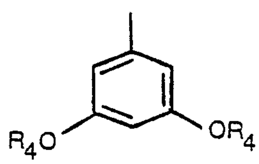 , 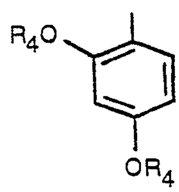 , 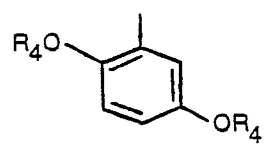

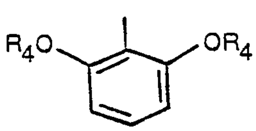 , 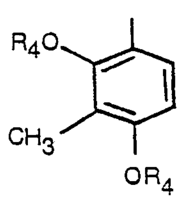 , 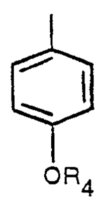 , 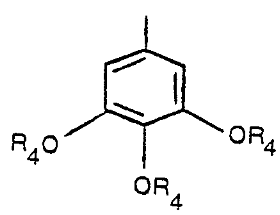 , ou

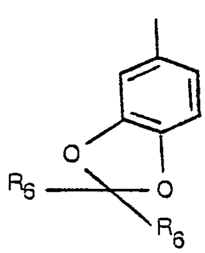 ;

où

$R_4$ est un groupe alkyle en $C_1$—$C_8$, $R_5$ est un groupe benzyle, et $R_6$ est l'hydrogène ou un groupe méthyle, avec la condition que lorsque R est $R_3CH_2$— où $R_3$ est

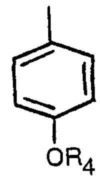

p ne peut pas être égal à 2;
qui consiste à traiter un halogénure de cétimine-magnésium de formule:

$$R—\overset{\overset{\displaystyle CF_pH_{3-p}}{|}}{C}=N—MgZ; \qquad II$$

dans laquelle R et p ont la signification définie ci-dessus et Z est chlorure, bromure ou iodure; avec le cyanure d'hydrogène ou avec un cyanure de métal alcalin ou le cyanure d'ammonium et une source de protons.